(19) Europäisches Patentamt
European Patent Office
Office européen des brevets

(11) EP 4 356 727 A2

(12) EUROPEAN PATENT APPLICATION

(43) Date of publication:
**24.04.2024 Bulletin 2024/17**

(21) Application number: **24160383.6**

(22) Date of filing: **05.04.2019**

(51) International Patent Classification (IPC):
***A01K 67/027*** *(2024.01)*

(52) Cooperative Patent Classification (CPC):
**A61K 48/005; A01K 67/0278; C07K 14/4728; C12N 15/86;** A01K 2217/072; A01K 2227/105; A01K 2267/0306; A61K 48/0058; C12N 2750/14143; C12N 2830/008; C12N 2830/48; C12N 2840/203

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **05.04.2018 IT 201800004253**

(62) Document number(s) of the earlier application(s) in accordance with Art. 76 EPC:
**19724606.9 / 3 773 744**

(71) Applicant: **Istituti Clinici Scientifici Maugeri S.p.A. S.B.**
**27100 Pavia (IT)**

(72) Inventors:
- **PRIORI, Silvia G.**
  **20154 Milano (MI) (IT)**
- **MAZZANTI, Andrea**
  **27016 Lardirago (PV) (IT)**
- **DENEGRI, Marco**
  **27049 Stradella (PV) (IT)**
- **NAPOLITANO, Carlo**
  **20154 Milano (MI) (IT)**

(74) Representative: **Casci, Tamara et al**
**Barzanò & Zanardo Milano S.p.A.**
**Via Borgonuovo, 10**
**20121 Milano (IT)**

Remarks:
- The complete document including Reference Table(s) and the Sequence Listing(s) can be downloaded from the EPO website
- This application was filed on 28.02.2024 as a divisional application to the application mentioned under INID code 62.

# (54) COMPOSITIONS AND METHODS FOR THE TREATMENT OF DOMINANTLY-INHERITED CATECHOLAMINERGIC POLYMORPHIC VENTRICULAR TACHYCARDIA

(57) The disclosure features compositions and methods for the treatment of catecholaminergic polymorphic ventricular tachycardia (CPVT), particularly forms of the disease that are inherited in an autosomal dominant manner, by way of calsequestrin 2 (CASQ2) gene therapy. The compositions and methods described herein can be used to treat CPVT caused by mutations in, for example, ryanodine receptor 2 and calmodulin, such as calmodulin 1 (CALM1) and CALMS. The disclosure provides a variety of vectors that can be used for the delivery of a CASQ2 transgene to a patient, such as a human patient suffering from autosomal dominant CPVT, including adeno-associated virus vectors, among others.

EP 4 356 727 A2

## Description

### Field of the Invention

The invention relates to the field of nucleic acid biotechnology and provides compositions and methods for treating heritable genetic disorders.

### Background of the Invention

Catecholaminergic polymorphic ventricular tachycardia (CPVT) is characterized by dysregulation of calcium signaling in cardiac cells. Patients having dominant CPVT often exhibit excessive or dysregulated release of calcium from the sarcoplasmic reticulum of cardiac myocytes, particularly during the diastole. This release of calcium can trigger a series of signaling events terminating in an action potential that may give rise to an extrasystolic heartbeat. This, in turn, can lead to arrhythmia, which, if left untreated, can be life-threatening. There is a paucity of strategies available for successfully ameliorating the symptoms of CPVT, and there remains a need for curative therapeutics for this disease.

### Summary of the Invention

Described herein are compositions and methods for the treatment of a dominantly-inherited form of catecholaminergic polymorphic ventricular tachycardia (CPVT) in a patient, such as a human patient. The compositions and methods of the disclosure pertain to the delivery of a gene encoding a functional calsequestrin 2 (CASQ2) protein to a patient suffering from dominant CPVT. For example, using the compositions and methods described herein, a transgene encoding a functional CASQ2 protein may be delivered to a patient by way of viral gene therapy. The transgene may be delivered in such a way as to facilitate expression of CASQ2 in a cell of the patient, such as a cardiac cell (e.g., a cardiac myocyte). The disclosure is based, in part, on the discovery that delivery of a gene encoding CASQ2 can ameliorate CPVT in a patient that already expresses functional CASQ2 protein, and rather suffers from one or more harmful mutations in another calcium-regulating protein. For example, the patient may exhibit one or more of a variety of mutations that give rise to dominant CPVT, such as a gain-of-function mutation in a gene encoding ryanodine receptor 2 (RYR2) and/or a mutation in a gene encoding a calmodulin, such as calmodulin 1 (CALM1) or calmodulin 3 (CALM3). Using the compositions and methods described herein, cardiac calcium regulation may be restored in patients having dominant CPVT, despite the presence of mutations in various calcium-modulating proteins and without the need to provide functional versions of mutated endogenous genes.

In a first aspect, the invention features a method of treating or preventing dominant CPVT in a patient (e.g., a mammal, such as a human patient, in need thereof) by administering to the patient a therapeutically effective amount of a composition containing a transgene encoding CASQ2.

In another aspect, the disclosure features a method of reducing arrhythmia in a patient (e.g., a mammal, such as a human patient) diagnosed as having dominant CPVT by administering to the patient a therapeutically effective amount of a composition containing a transgene encoding CASQ2. For example, the disclosure features a method of reducing the quantity of arrhythmia events in a patient (e.g., a mammal, such as a human patient) diagnosed as having dominant CPVT by administering to the patient a therapeutically effective amount of a composition containing a transgene encoding CASQ2. The disclosure additionally features a method of reducing the frequency of arrhythmia events in a patient (e.g., a mammal, such as a human patient) diagnosed as having dominant CPVT by administering to the patient a therapeutically effective amount of a composition containing a transgene encoding CASQ2.

In another aspect, the disclosure features a method of restoring calcium homeostasis in the heart of a patient (e.g., a mammal, such as a human patient) diagnosed as having dominant CPVT by administering to the patient a therapeutically effective amount of a composition containing a transgene encoding CASQ2.

In an additional aspect, the disclosure features a method of reducing spontaneous calcium releases, for example, in a cardiac myocyte, in a patient (e.g., a mammal, such as a human patient) diagnosed as having dominant CPVT by administering to the patient a therapeutically effective amount of a composition containing a transgene encoding CASQ2.

In a further aspect, the disclosure features a method of reducing delayed afterdepolarizations in a patient (e.g., a mammal, such as a human patient) diagnosed as having dominant CPVT by administering to the patient a therapeutically effective amount of a composition containing a transgene encoding CASQ2.

In some embodiments of any of the above aspects of the disclosure, the patient exhibits a gain-of-function mutation in an endogenous gene encoding RYR2. For example, the mutation in RYR2 may result in an increase in cytosolic calcium release in a cardiac myocyte that is contacted with a RYR2 agonist relative to a cardiac myocyte that is not contacted with the RYR2 agonist. Particularly, the mutation in RYR2 may be R4497C, N4104K, N4895D, R176Q, G178A, R420W, L433P, P1067S, S2246L, G2273R, F2307L, E2311D, L2344P, R2474S, T2504M, K3717R, L3778F, G3926D, G3946S, Q4159P, V4319-K4324dup, R4651I, V4771I, F4851L, A4860G, I4867M, P4902S, and/or R4959Q.

For example, the patient may have a combination of two or more of the mutations in RYR2 set forth above. In some embodiments, the patient has the R4497C mutation and a N4104K, N4895D, R176Q, G178A, R420W, L433P, P1067S, S2246L, G2273R, F2307L, E2311D, L2344P, R2474S, T2504M, K3717R, L3778F, G3926D, G3946S, Q4159P, V4319-K4324dup, R4651I, V4771I, F4851L, A4860G, I4867M, P4902S, or R4959Q mutation.

In some embodiments, the patient has the N4104K mutation and one or more of the R4497C, N4895D, R176Q, G178A, R420W, L433P, P1067S, S2246L, G2273R, F2307L, E2311D, L2344P, R2474S, T2504M, K3717R, L3778F, G3926D, G3946S, Q4159P, V4319-K4324dup, R4651I, V4771I, F4851L, A4860G, I4867M, P4902S, and R4959Q mutations.

In some embodiments, the patient has the N4895D mutation and one or more of the R4497C, N4104K, R176Q, G178A, R420W, L433P, P1067S, S2246L, G2273R, F2307L, E2311D, L2344P, R2474S, T2504M, K3717R, L3778F, G3926D, G3946S, Q4159P, V4319-K4324dup, R4651I, V4771I, F4851L, A4860G, I4867M, P4902S, and R4959Q mutations.

In some embodiments, the patient has the R176Q mutation and one or more of the R4497C, N4104K, N4895D, G178A, R420W, L433P, P1067S, S2246L, G2273R, F2307L, E2311D, L2344P, R2474S, T2504M, K3717R, L3778F, G3926D, G3946S, Q4159P, V4319-K4324dup, R4651I, V4771I, F4851L, A4860G, I4867M, P4902S, and R4959Q mutations.

In some embodiments, the patient has the G178A mutation and one or more of the R4497C, N4104K, N4895D, R176Q, R420W, L433P, P1067S, S2246L, G2273R, F2307L, E2311D, L2344P, R2474S, T2504M, K3717R, L3778F, G3926D, G3946S, Q4159P, V4319-K4324dup, R4651I, V4771I, F4851L, A4860G, I4867M, P4902S, and R4959Q mutations.

In some embodiments, the patient has the R420W mutation and one or more of the R4497C, N4104K, N4895D, R176Q, G178A, L433P, P1067S, S2246L, G2273R, F2307L, E2311D, L2344P, R2474S, T2504M, K3717R, L3778F, G3926D, G3946S, Q4159P, V4319-K4324dup, R4651I, V4771I, F4851L, A4860G, I4867M, P4902S, and R4959Q mutations.

In some embodiments, the patient has the G178A mutation and one or more of the R4497C, N4104K, N4895D, R176Q, R420W, L433P, P1067S, S2246L, G2273R, F2307L, E2311D, L2344P, R2474S, T2504M, K3717R, L3778F, G3926D, G3946S, Q4159P, V4319-K4324dup, R4651I, V4771I, F4851L, A4860G, I4867M, P4902S, and R4959Q mutations.

In some embodiments, the patient has the L433P mutation and one or more of the R4497C, N4104K, N4895D, R176Q, R420W, G178A, P1067S, S2246L, G2273R, F2307L, E2311D, L2344P, R2474S, T2504M, K3717R, L3778F, G3926D, G3946S, Q4159P, V4319-K4324dup, R4651I, V4771I, F4851L, A4860G, I4867M, P4902S, and R4959Q mutations.

In some embodiments, the patient has the P1067S mutation and one or more of the R4497C, N4104K, N4895D, R176Q, R420W, G178A, L433P, S2246L, G2273R, F2307L, E2311D, L2344P, R2474S, T2504M, K3717R, L3778F, G3926D, G3946S, Q4159P, V4319-K4324dup, R4651I, V4771I, F4851L, A4860G, I4867M, P4902S, and R4959Q mutations.

In some embodiments, the patient has the S2246L mutation and one or more of the R4497C, N4104K, N4895D, R176Q, R420W, G178A, L433P, P1067S, G2273R, F2307L, E2311D, L2344P, R2474S, T2504M, K3717R, L3778F, G3926D, G3946S, Q4159P, V4319-K4324dup, R4651I, V4771I, F4851L, A4860G, I4867M, P4902S, and R4959Q mutations.

In some embodiments, the patient has the G2273R mutation and one or more of the R4497C, N4104K, N4895D, R176Q, R420W, G178A, L433P, P1067S, S2246L, F2307L, E2311D, L2344P, R2474S, T2504M, K3717R, L3778F, G3926D, G3946S, Q4159P, V4319-K4324dup, R4651I, V4771I, F4851L, A4860G, I4867M, P4902S, and R4959Q mutations.

In some embodiments, the patient has the F2307L mutation and one or more of the R4497C, N4104K, N4895D, R176Q, R420W, G178A, L433P, P1067S, S2246L, G2273R, E2311D, L2344P, R2474S, T2504M, K3717R, L3778F, G3926D, G3946S, Q4159P, V4319-K4324dup, R4651I, V4771I, F4851L, A4860G, I4867M, P4902S, and R4959Q mutations.

In some embodiments, the patient has the E2311D mutation and one or more of the R4497C, N4104K, N4895D, R176Q, R420W, G178A, L433P, P1067S, S2246L, G2273R, F2307L, L2344P, R2474S, T2504M, K3717R, L3778F, G3926D, G3946S, Q4159P, V4319-K4324dup, R4651I, V4771I, F4851L, A4860G, I4867M, P4902S, and R4959Q mutations.

In some embodiments, the patient has the L2344P mutation and one or more of the R4497C, N4104K, N4895D, R176Q, R420W, G178A, L433P, P1067S, S2246L, G2273R, F2307L, E2311D, R2474S, T2504M, K3717R, L3778F, G3926D, G3946S, Q4159P, V4319-K4324dup, R4651I, V4771I, F4851L, A4860G, I4867M, P4902S, and R4959Q mutations.

In some embodiments, the patient has the R2474S mutation and one or more of the R4497C, N4104K, N4895D, R176Q, R420W, G178A, L433P, P1067S, S2246L, G2273R, F2307L, E2311D, L2344P, T2504M, K3717R, L3778F, G3926D, G3946S, Q4159P, V4319-K4324dup, R4651I, V4771I, F4851L, A4860G, I4867M, P4902S, and R4959Q mutations.

In some embodiments, the patient has the T2504M mutation and one or more of the R4497C, N4104K, N4895D, R176Q, R420W, G178A, L433P, P1067S, S2246L, G2273R, F2307L, E2311D, L2344P, R2474S, K3717R, L3778F, G3926D, G3946S, Q4159P, V4319-K4324dup, R4651I, V4771I, F4851L, A4860G, I4867M, P4902S, and R4959Q mutations.

In some embodiments, the patient has the K3717R mutation and one or more of the R4497C, N4104K, N4895D, R176Q, R420W, G178A, L433P, P1067S, S2246L, G2273R, F2307L, E2311D, L2344P, R2474S, T2504M, L3778F, G3926D, G3946S, Q4159P, V4319-K4324dup, R4651I, V4771I, F4851L, A4860G, I4867M, P4902S, and R4959Q mutations.

In some embodiments, the patient has the L3778F mutation and one or more of the R4497C, N4104K, N4895D, R176Q, R420W, G178A, L433P, P1067S, S2246L, G2273R, F2307L, E2311D, L2344P, R2474S, T2504M, K3717R, G3926D, G3946S, Q4159P, V4319-K4324dup, R4651I, V4771I, F4851L, A4860G, I4867M, P4902S, and R4959Q mutations.

In some embodiments, the patient has the G3926D mutation and one or more of the R4497C, N4104K, N4895D, R176Q, R420W, G178A, L433P, P1067S, S2246L, G2273R, F2307L, E2311D, L2344P, R2474S, T2504M, K3717R, L3778F, G3946S, Q4159P, V4319-K4324dup, R4651I, V4771I, F4851L, A4860G, I4867M, P4902S, and R4959Q mutations.

In some embodiments, the patient has the G3946S mutation and one or more of the R4497C, N4104K, N4895D, R176Q, R420W, G178A, L433P, P1067S, S2246L, G2273R, F2307L, E2311D, L2344P, R2474S, T2504M, K3717R, L3778F, G3926D, Q4159P, V4319-K4324dup, R4651I, V4771I, F4851L, A4860G, I4867M, P4902S, and R4959Q mutations.

In some embodiments, the patient has the Q4159P mutation and one or more of the R4497C, N4104K, N4895D, R176Q, R420W, G178A, L433P, P1067S, S2246L, G2273R, F2307L, E2311D, L2344P, R2474S, T2504M, K3717R, L3778F, G3926D, G3946S, V4319-K4324dup, R4651I, V4771I, F4851L, A4860G, I4867M, P4902S, and R4959Q mutations.

In some embodiments, the patient has the V4319-K4324dup mutation and one or more of the R4497C, N4104K, N4895D, R176Q, R420W, G178A, L433P, P1067S, S2246L, G2273R, F2307L, E2311D, L2344P, R2474S, T2504M, K3717R, L3778F, G3926D, G3946S, Q4159P, R4651I, V4771I, F4851L, A4860G, I4867M, P4902S, and R4959Q mutations.

In some embodiments, the patient has the R4651I mutation and one or more of the R4497C, N4104K, N4895D, R176Q, R420W, G178A, L433P, P1067S, S2246L, G2273R, F2307L, E2311D, L2344P, R2474S, T2504M, K3717R, L3778F, G3926D, G3946S, Q4159P, V4319-K4324dup, V4771I, F4851L, A4860G, I4867M, P4902S, and R4959Q mutations.

In some embodiments, the patient has the V4771I mutation and one or more of the R4497C, N4104K, N4895D, R176Q, R420W, G178A, L433P, P1067S, S2246L, G2273R, F2307L, E2311D, L2344P, R2474S, T2504M, K3717R, L3778F, G3926D, G3946S, Q4159P, V4319-K4324dup, R4651I, F4851L, A4860G, I4867M, P4902S, and R4959Q mutations.

In some embodiments, the patient has the F4851L mutation and one or more of the R4497C, N4104K, N4895D, R176Q, R420W, G178A, L433P, P1067S, S2246L, G2273R, F2307L, E2311D, L2344P, R2474S, T2504M, K3717R, L3778F, G3926D, G3946S, Q4159P, V4319-K4324dup, R4651I, V4771I, A4860G, I4867M, P4902S, and R4959Q mutations.

In some embodiments, the patient has the A4860G mutation and one or more of the R4497C, N4104K, N4895D, R176Q, R420W, G178A, L433P, P1067S, S2246L, G2273R, F2307L, E2311D, L2344P, R2474S, T2504M, K3717R, L3778F, G3926D, G3946S, Q4159P, V4319-K4324dup, R4651I, V4771I, F4851L, I4867M, P4902S, and R4959Q mutations.

In some embodiments, the patient has the I4867M mutation and one or more of the R4497C, N4104K, N4895D, R176Q, R420W, G178A, L433P, P1067S, S2246L, G2273R, F2307L, E2311D, L2344P, R2474S, T2504M, K3717R, L3778F, G3926D, G3946S, Q4159P, V4319-K4324dup, R4651I, V4771I, F4851L, A4860G, P4902S, and R4959Q mutations.

In some embodiments, the patient has the P4902S mutation and one or more of the R4497C, N4104K, N4895D, R176Q, R420W, G178A, L433P, P1067S, S2246L, G2273R, F2307L, E2311D, L2344P, R2474S, T2504M, K3717R, L3778F, G3926D, G3946S, Q4159P, V4319-K4324dup, R4651I, V4771I, F4851L, A4860G, I4867M, and R4959Q mutations.

In some embodiments, the patient has the R4959Q mutation and one or more of the R4497C, N4104K, N4895D, R176Q, R420W, G178A, L433P, P1067S, S2246L, G2273R, F2307L, E2311D, L2344P, R2474S, T2504M, K3717R, L3778F, G3926D, G3946S, Q4159P, V4319-K4324dup, R4651I, V4771I, F4851L, A4860G, I4867M, and P4902S mutations.

In some embodiments, the patient exhibits a mutation in an endogenous gene encoding a calmodulin. For example, the patient may exhibit a mutation in CALM1, such as a mutation that reduces CALM1 inhibition of calcium release from the sarcoplasmic reticulum of a cardiac myocyte.

In some embodiments, the CALM1 harboring the mutation directly activates calcium release from the sarcoplasmic reticulum of a cardiac myocyte. For example, the mutation may increase the stability of an open conformation of RYR2 in a CALM1-RYR2 complex present in an aqueous solution having a concentration of $Ca^{2+}$ of from about 50 nM to about 5 $\mu$M (e.g., a concentration of $Ca^{2+}$ of about 50 nM, 100 nM, 150 nM, 200 nM, 250 nM, 300 nM, 350 nM, 400 nM, 450 nM, 500 nM, 550 nM, 600 nM, 650 nM, 700 nM, 750 nM, 800 nM, 850 nM, 900 nM, 950 nM, 1 $\mu$M, 2 $\mu$M, 3 $\mu$M, 4 $\mu$M, or 5 $\mu$M). In some embodiments, the mutation in CALM1 is N97S or N53I.

In some embodiments, the patient exhibits a mutation in CALM3, such as a mutation that results in the direct activation of calcium release from the sarcoplasmic reticulum in a cardiac myocyte. In some embodiments, the mutation in CALM3 is A103V.

In some embodiments, the mutation in RYR2 or a calmodulin (e.g., CALM1 and/or CALM3) is heterozygous.

In some embodiments (e.g., in which a human patient is treated), the CASQ2 transgene encodes a protein having an amino acid sequence that is at least 85% identical to the amino acid sequence of SEQ ID NO: 1 (e.g., 85%, 86%, 87%, 88%, 89%, 90%, 91%, 92%, 93%, 94%, 95%, 96%, 97%, 98%, 99%, 99.9%, or 100% identical to the amino acid sequence of SEQ ID NO: 1). The CASQ2 transgene may encode a protein having one or more conservative amino acid substitutions relative to the amino acid sequence of SEQ ID NO: 1. For example, the CASQ2 transgene may encode a protein having up to 50 conservative amino acid substitutions (e.g., 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50 conservative amino acid substitutions) relative to the amino acid sequence of SEQ ID NO: 1. In some embodiments, the CASQ2 transgene encodes a protein having up to 25 conservative amino acid substitutions (e.g., 1, 2, 3, 4, 5, 6, 7, 8, 9, 10, 11, 12, 13, 14, 15, 16, 17, 18, 19, 20, 21, 22, 23, 24, or 25 conservative amino acid substitutions) relative to the amino acid sequence of SEQ ID NO: 1. In some embodiments, the CASQ2 transgene encodes a protein having up to 10 conservative amino acid substitutions (e.g., 1, 2, 3, 4, 5, 6, 7, 8, 9, or 10 conservative amino acid substitutions) relative to the amino acid sequence of SEQ ID NO: 1. In some embodiments, the CASQ2 transgene encodes having an amino acid sequence that differs from that of SEQ ID NO: 1 only by way of conservative amino acid substitutions.

In some embodiments (e.g., in which a human patient is treated), the CASQ2 transgene encodes a protein having one or more nonconservative amino acid substitutions relative to the amino acid sequence of SEQ ID NO: 1. For example, the CASQ2 transgene may encode a protein having up to 50 nonconservative amino acid substitutions (e.g., 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50 nonconservative amino acid substitutions) relative to the amino acid sequence of SEQ ID NO: 1. In some embodiments, the CASQ2 transgene encodes a protein having up to 25 nonconservative amino acid substitutions (e.g., 1, 2, 3, 4, 5, 6, 7, 8, 9, 10, 11, 12, 13, 14, 15, 16, 17, 18, 19, 20, 21, 22, 23, 24, or 25 conservative amino acid substitutions) relative to the amino acid sequence of SEQ ID NO: 1. In some embodiments, the CASQ2 transgene encodes a protein having up to 10 nonconservative amino acid substitutions (e.g., 1, 2, 3, 4, 5, 6, 7, 8, 9, or 10 conservative amino acid substitutions) relative to the amino acid sequence of SEQ ID NO: 1.

In some embodiments, the CASQ2 transgene encodes protein having an amino acid sequence that is at least 85% identical to the amino acid sequence of SEQ ID NO: 3 (e.g., 85%, 86%, 87%, 88%, 89%, 90%, 91%, 92%, 93%, 94%, 95%, 96%, 97%, 98%, 99%, 99.9%, or 100% identical to the amino acid sequence of SEQ ID NO: 3). The CASQ2 transgene may encode a protein having one or more conservative amino acid substitutions relative to the amino acid sequence of SEQ ID NO: 3. For example, the CASQ2 transgene may encode a protein having up to 50 conservative amino acid substitutions (e.g., 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50 conservative amino acid substitutions) relative to the amino acid sequence of SEQ ID NO: 3. In some embodiments, the CASQ2 transgene encodes a protein having up to 25 conservative amino acid substitutions (e.g., 1, 2, 3, 4, 5, 6, 7, 8, 9, 10, 11, 12, 13, 14, 15, 16, 17, 18, 19, 20, 21, 22, 23, 24, or 25 conservative amino acid substitutions) relative to the amino acid sequence of SEQ ID NO: 3. In some embodiments, the CASQ2 transgene encodes a protein having up to 10 conservative amino acid substitutions (e.g., 1, 2, 3, 4, 5, 6, 7, 8, 9, or 10 conservative amino acid substitutions) relative to the amino acid sequence of SEQ ID NO: 3. In some embodiments, the CASQ2 transgene encodes having an amino acid sequence that differs from that of SEQ ID NO: 3 only by way of conservative amino acid substitutions.

In some embodiments, the CASQ2 transgene encodes a protein having one or more nonconservative amino acid substitutions relative to the amino acid sequence of SEQ ID NO: 3. For example, the CASQ2 transgene may encode a protein having up to 50 nonconservative amino acid substitutions (e.g., 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50 nonconservative amino acid substitutions) relative to the amino acid sequence of SEQ ID NO: 3. In some embodiments, the CASQ2 transgene encodes a protein having up to 25 nonconservative amino acid substitutions (e.g., 1, 2, 3, 4, 5, 6, 7, 8, 9, 10, 11, 12, 13, 14, 15, 16, 17, 18, 19, 20, 21, 22, 23, 24, or 25 conservative amino acid substitutions) relative to the amino acid sequence of SEQ ID NO: 3. In some embodiments, the CASQ2 transgene encodes a protein having up to 10 nonconservative amino acid substitutions (e.g., 1, 2, 3, 4, 5, 6, 7, 8, 9, or 10 conservative amino acid substitutions) relative to the amino acid sequence of SEQ ID NO: 3.

In some embodiments, (e.g., in which a human patient is treated), the CASQ2 transgene has a nucleic acid sequence that is at least 85% identical to the nucleic acid sequence of SEQ ID NO: 2 (e.g., 85%, 86%, 87%, 88%, 89%, 90%, 91%, 92%, 93%, 94%, 95%, 96%, 97%, 98%, 99%, 99.9%, or 100% identical to the nucleic acid sequence of SEQ ID NO: 2).

In some embodiments, the CASQ2 transgene has a nucleic acid sequence that is at least 85% identical to the nucleic acid sequence of SEQ ID NO: 4 (e.g., 85%, 86%, 87%, 88%, 89%, 90%, 91%, 92%, 93%, 94%, 95%, 96%, 97%, 98%, 99%, 99.9%, or 100% identical to the nucleic acid sequence of SEQ ID NO: 4).

In some embodiments, the composition containing the CASQ2 transgene is a vector, such as a viral vector. The viral vector may be, for example, an adeno-associated virus (AAV), adenovirus, lentivirus, retrovirus, poxvirus, baculovirus, herpes simplex virus, vaccinia virus, or a synthetic virus (e.g., a chimeric virus, mosaic virus, or pseudotyped virus, and/or a virus containing a foreign protein, synthetic polymer, nanoparticle, and/or small molecule). In some embodiments, the viral vector is an AAV vector, such as AAV1, AAV2, AAV3, AAV4, AAV5, AAV6, AAV7, AAV8, AAV9, or AAVrh74 serotype vector. The AAV vector may be pseudotyped. In some embodiments, the pseudotyped AAV vector is AAV2/8 or AV2/9.

In some embodiments, the composition is a vector (e.g., a viral vector, such as an AAV vector described above, e.g.an AAV1, AAV2, AAV3, AAV4, AAV5, AAV6, AAV7, AAV8, AAV9, AAVrh74, AAV2/8, or AV2/9, vector), and the vector is administered to the patient at a dose of from about $1 \times 10^6$ genome copies (GC)/kg to about $1 \times 10^{18}$ GC/kg (e.g., at a dose of about $1 \times 10^6$ GC/kg, $2 \times 10^6$ GC/kg, $3 \times 10^6$ GC/kg, $4 \times 10^6$ GC/kg, $5 \times 10^6$ GC/kg, $6 \times 10^6$ GC/kg, $7 \times 10^6$ GC/kg, $8 \times 10^6$ GC/kg, 9x $10^6$ GC/kg, $1 \times 10^7$ GC/kg, $2 \times 10^7$ GC/kg, $3 \times 10^7$ GC/kg, $4 \times 10^7$ GC/kg, $5 \times 10^7$ GC/kg, $6 \times 10^7$ GC/kg, $7 \times 10^7$ GC/kg, $8 \times 10^7$ GC/kg, $9 \times 10^7$ GC/kg, $1 \times 10^8$ GC/kg, $2 \times 10^8$ GC/kg, $3 \times 10^8$ GC/kg, $4 \times 10^8$ GC/kg, $5 \times 10^8$ GC/kg, $6 \times 10^8$ GC/kg, $7 \times 10^8$ GC/kg, $8 \times 10^8$ GC/kg, $9 \times 10^8$ GC/kg, $1 \times 10^9$ GC/kg, $2 \times 10^9$ GC/kg, $3 \times 10^9$ GC/kg, $4 \times 10^9$ GC/kg, $5 \times 10^9$ GC/kg, $6 \times 10^9$ GC/kg, $7 \times 10^9$ GC/kg, $8 \times 10^9$ GC/kg, $9 \times 10^9$ GC/kg, $1 \times 10^{10}$ GC/kg, $2 \times 10^{10}$ GC/kg, $3 \times 10^{10}$ GC/kg, $4 \times 10^{10}$ GC/kg, $5 \times 10^{10}$ GC/kg, $6 \times 10^{10}$ GC/kg, $7 \times 10^{10}$ GC/kg, $8 \times 10^{10}$ GC/kg, $9 \times 10^{10}$ GC/kg, $1 \times 10^{11}$ GC/kg, $2 \times 10^{11}$ GC/kg, $3 \times 10^{11}$ GC/kg, $4 \times 10^{11}$ GC/kg, $5 \times 10^{11}$ GC/kg, $6 \times 10^{11}$ GC/kg, $7 \times 10^{11}$ GC/kg, $8 \times 10^{11}$ GC/kg, $9 \times 10^{11}$ GC/kg, $1 \times 10^{12}$ GC/kg, $2 \times 10^{12}$ GC/kg, $3 \times 10^{12}$ GC/kg, $4 \times 10^{12}$ GC/kg, $5 \times 10^{12}$ GC/kg, $6 \times 10^{12}$ GC/kg, $7 \times 10^{12}$ GC/kg, $8 \times 10^{12}$ GC/kg, $9 \times 10^{12}$ GC/kg, $1 \times 10^{13}$ GC/kg, $2 \times 10^{13}$ GC/kg, $3 \times 10^{13}$ GC/kg, $4 \times 10^{13}$ GC/kg, $5 \times 10^{13}$ GC/kg, $6 \times 10^{13}$ GC/kg, $7 \times 10^{13}$ GC/kg, $8 \times 10^{13}$ GC/kg, $9 \times 10^{13}$ GC/kg, $1 \times 10^{14}$ GC/kg, $2 \times 10^{14}$ GC/kg, $3 \times 10^{14}$ GC/kg, $4 \times 10^{14}$ GC/kg, $5 \times 10^{14}$ GC/kg, $6 \times 10^{14}$ GC/kg, $7 \times 10^{14}$ GC/kg, $8 \times 10^{14}$ GC/kg, $9 \times 10^{14}$ GC/kg, $1 \times 10^{15}$ GC/kg, $2 \times 10^{15}$ GC/kg, $3 \times 10^{15}$ GC/kg, $4 \times 10^{15}$ GC/kg, $5 \times 10^{15}$ GC/kg, $6 \times 10^{15}$ GC/kg, $7 \times 10^{15}$ GC/kg, $8 \times 10^{15}$ GC/kg, $9 \times 10^{15}$ GC/kg, $1 \times 10^{16}$ GC/kg, $2 \times 10^{16}$ GC/kg, $3 \times 10^{16}$ GC/kg, $4 \times 10^{16}$ GC/kg, $5 \times 10^{16}$ GC/kg, $6 \times 10^{16}$ GC/kg, $7 \times 10^{16}$ GC/kg, $8 \times 10^{16}$ GC/kg, $9 \times 10^{16}$ GC/kg, $1 \times 10^{17}$ GC/kg, $2 \times 10^{17}$ GC/kg, $3 \times 10^{17}$ GC/kg, $4 \times 10^{17}$ GC/kg, $5 \times 10^{17}$ GC/kg, $6 \times 10^{17}$ GC/kg, $7 \times 10^{17}$ GC/kg, $8 \times 10^{17}$ GC/kg, $9 \times 10^{17}$ GC/kg, or $1 \times 10^{18}$ GC/kg). In some embodiments, the vector is administered to the patient at a dose of from about $1 \times 10^8$ GC/kg to about $1 \times 10^{16}$ GC/kg (e.g., at a dose of about $1 \times 10^8$ GC/kg, $2 \times 10^8$ GC/kg, $3 \times 10^8$ GC/kg, $4 \times 10^8$ GC/kg, $5 \times 10^8$ GC/kg, $6 \times 10^8$ GC/kg, $7 \times 10^8$ GC/kg, $8 \times 10^8$ GC/kg, $9 \times 10^8$ GC/kg, $1 \times 10^9$ GC/kg, $2 \times 10^9$ GC/kg, $3 \times 10^9$ GC/kg, $4 \times 10^9$ GC/kg, $5 \times 10^9$ GC/kg, $6 \times 10^9$ GC/kg, $7 \times 10^9$ GC/kg, $8 \times 10^9$ GC/kg, $9 \times 10^9$GC/kg, $1 \times 10^{10}$ GC/kg, $2 \times 10^{10}$ GC/kg, $3 \times 10^{10}$ GC/kg, $4 \times 10^{10}$ GC/kg, $5 \times 10^{10}$ GC/kg, $6 \times 10^{10}$ GC/kg, $7 \times 10^{10}$ GC/kg, $8 \times 10^{10}$ GC/kg, $9 \times 10^{10}$ GC/kg, $1 \times 10^{11}$ GC/kg, $2 \times 10^{11}$ GC/kg, $3 \times 10^{11}$ GC/kg, $4 \times 10^{11}$ GC/kg, $5 \times 10^{11}$ GC/kg, $6 \times 10^{11}$ GC/kg, $7 \times 10^{11}$ GC/kg, $8 \times 10^{11}$ GC/kg, $9 \times 10^{11}$ GC/kg, $1 \times 10^{12}$ GC/kg, $2 \times 10^{12}$ GC/kg, $3 \times 10^{12}$ GC/kg, $4 \times 10^{12}$ GC/kg, $5 \times 10^{12}$ GC/kg, $6 \times 10^{12}$ GC/kg, $7 \times 10^{12}$ GC/kg, $8 \times 10^{12}$ GC/kg, $9 \times 10^{12}$ GC/kg, $1 \times 10^{13}$ GC/kg, $2 \times 10^{13}$ GC/kg, $3 \times 10^{13}$ GC/kg, $4 \times 10^{13}$ GC/kg, $5 \times 10^{13}$ GC/kg, $6 \times 10^{13}$ GC/kg, $7 \times 10^{13}$ GC/kg, $8 \times 10^{13}$ GC/kg, $9 \times 10^{13}$ GC/kg, $1 \times 10^{14}$ GC/kg, $2 \times 10^{14}$ GC/kg, $3 \times 10^{14}$ GC/kg, $4 \times 10^{14}$ GC/kg, $5 \times 10^{14}$ GC/kg, $6 \times 10^{14}$ GC/kg, $7 \times 10^{14}$ GC/kg, $8 \times 10^{14}$ GC/kg, $9 \times 10^{14}$ GC/kg, $1 \times 10^{15}$ GC/kg, $2 \times 10^{15}$ GC/kg, $3 \times 10^{15}$ GC/kg, $4 \times 10^{15}$ GC/kg, $5 \times 10^{15}$ GC/kg, $6 \times 10^{15}$ GC/kg, $7 \times 10^{15}$ GC/kg, $8 \times 10^{15}$ GC/kg, $9 \times 10^{15}$ GC/kg, or $1 \times 10^{16}$ GC/kg). In some embodiments, the vector is administered to the patient at a dose of from about $1 \times 10^{10}$ GC/kg to about $1 \times 10^{14}$ GC/kg (e.g., at a dose of about $1 \times 10^{10}$ GC/kg, $2 \times 10^{10}$ GC/kg, $3 \times 10^{10}$ GC/kg, $4 \times 10^{10}$ GC/kg, $5 \times 10^{10}$ GC/kg, $6 \times 10^{10}$ GC/kg, $7 \times 10^{10}$ GC/kg, $8 \times 10^{10}$ GC/kg, $9 \times 10^{10}$ GC/kg, $1 \times 10^{11}$ GC/kg, $2 \times 10^{11}$ GC/kg, $3 \times 10^{11}$ GC/kg, $4 \times 10^{11}$ GC/kg, $5 \times 10^{11}$ GC/kg, $6 \times 10^{11}$ GC/kg, $7 \times 10^{11}$ GC/kg, $8 \times 10^{11}$ GC/kg, $9 \times 10^{11}$ GC/kg, $1 \times 10^{12}$ GC/kg, $2 \times 10^{12}$ GC/kg, $3 \times 10^{12}$ GC/kg, $4 \times 10^{12}$ GC/kg, $5 \times 10^{12}$ GC/kg, $6 \times 10^{12}$ GC/kg, $7 \times 10^{12}$ GC/kg, $8 \times 10^{12}$ GC/kg, $9 \times 10^{12}$ GC/kg, $1 \times 10^{13}$ GC/kg, $2 \times 10^{13}$ GC/kg, $3 \times 10^{13}$ GC/kg, $4 \times 10^{13}$ GC/kg, $5 \times 10^{13}$ GC/kg, $6 \times 10^{13}$ GC/kg, $7 \times 10^{13}$ GC/kg, $8 \times 10^{13}$ GC/kg, $9 \times 10^{13}$ GC/kg, or $1 \times 10^{14}$ GC/kg).

In some embodiments, the composition is a liposome, vesicle, synthetic vesicle, exosome, synthetic exosome, dendrimer, or nanoparticle.

In some embodiments, the transgene encoding CASQ2 is operably linked to a promoter that induces expression of the CASQ2 in a cardiac myocyte. For example, the promoter may be a desmin promoter, cytomegalovirus promoter, myosin light chain-2 promoter, alpha actin promoter, troponin 1 promoter, $Na^+/Ca^{2+}$ exchanger promoter, dystrophin promoter, creatine kinase promoter, alpha7 integrin promoter, brain natriuretic peptide promoter, alpha B-crystallin/small heat shock protein promoter, alpha myosin heavy chain promoter, or atrial natriuretic factor promoter. In some embodiments, the promoter is a desmin promoter.

In some embodiments, the desmin promoter contains a region having the nucleic acid sequence of nucleotides -984 to +76 with respect to the endogenous human desmin transcription start site, or a region that is at least 85% identical (e.g., 85%, 86%, 87%, 88%, 89%, 90%, 91%, 92%, 93%, 94%, 95%, 96%, 97%, 98%, 99%, or 100% identical) to this nucleic acid sequence). In some embodiments, the desmin promoter contains a region having the nucleic acid sequence of nucleotides -973 to -693 with respect to the endogenous human desmin transcription start site, or a region that is at least 85% identical (e.g., 85%, 86%, 87%, 88%, 89%, 90%, 91%, 92%, 93%, 94%, 95%, 96%, 97%, 98%, 99%, or 100% identical) to this nucleic acid sequence). In some embodiments, the desmin promoter contains a region having the nucleic acid sequence of nucleotides -984 to -644 with respect to the endogenous human desmin transcription start site, or a region that is at least 85% identical (e.g., 85%, 86%, 87%, 88%, 89%, 90%, 91%, 92%, 93%, 94%, 95%, 96%, 97%, 98%, 99%, or 100% identical) to this nucleic acid sequence). In some embodiments, the desmin promoter contains a region having the nucleic acid sequence of nucleotides -269 to +76 with respect to the endogenous human desmin transcription start site, or a region that is at least 85% identical (e.g., 85%, 86%, 87%, 88%, 89%, 90%, 91%, 92%, 93%, 94%, 95%, 96%, 97%, 98%, 99%, or 100% identical) to this nucleic acid sequence).

In some embodiments, the desmin promoter contains a first region having (i) the nucleic acid sequence of nucleotides -973 to -693 with respect to the endogenous human desmin transcription start site or (ii) at least 85% sequence identity (e.g., 85%, 86%, 87%, 88%, 89%, 90%, 91%, 92%, 93%, 94%, 95%, 96%, 97%, 98%, 99%, or 100% sequence identity) to the nucleic acid sequence of nucleotides -973 to -693 with respect to the endogenous human desmin transcription start site. The desmin promoter may further contain a second region having (i) the nucleic acid sequence of nucleotides -269 to +76 with respect to the endogenous human desmin transcription start site or (ii) at least 85% sequence identity (e.g., 85%, 86%, 87%, 88%, 89%, 90%, 91%, 92%, 93%, 94%, 95%, 96%, 97%, 98%, 99%, or 100% sequence identity) to the nucleic acid sequence of nucleotides -269 to +76 with respect to the endogenous human desmin transcription start site. In some embodiments, the first region is operably linked to the second region. For example, the first region may be joined directly to the second region, with or without intervening nucleotides. In some embodiments, the first region is located 5' of the second region (i.e., such that the 3' end of the first region is joined to the 5' end of the second region). In some embodiments, the first region is located 3' of the second region (i.e., such that the 5' end of the first region is joined to the 3' end of the second region).

In some embodiments, the desmin promoter contains a first region having (i) the nucleic acid sequence of nucleotides -984 to -644 with respect to the endogenous human desmin transcription start site or (ii) at least 85% sequence identity (e.g., 85%, 86%, 87%, 88%, 89%, 90%, 91%, 92%, 93%, 94%, 95%, 96%, 97%, 98%, 99%, or 100% sequence identity) to the nucleic acid sequence of nucleotides -984 to -644 with respect to the endogenous human desmin transcription start site. The desmin promoter may further contain a second region having (i) the nucleic acid sequence of nucleotides -269 to +76 with respect to the endogenous human desmin transcription start site or (ii) at least 85% sequence identity (e.g., 85%, 86%, 87%, 88%, 89%, 90%, 91%, 92%, 93%, 94%, 95%, 96%, 97%, 98%, 99%, or 100% sequence identity) to the nucleic acid sequence of nucleotides -269 to +76 with respect to the endogenous human desmin transcription start site. In some embodiments, the first region is operably linked to the second region. For example, the first region may be joined directly to the second region, with or without intervening nucleotides. In some embodiments, the first region is located 5' of the second region (i.e., such that the 3' end of the first region is joined to the 5' end of the second region). In some embodiments, the first region is located 3' of the second region (i.e., such that the 5' end of the first region is joined to the 3' end of the second region).

Exemplary desmin promoters that may be used in conjunction with the compositions and methods of the disclosure are described in Gao et al., Journal of Biological Chemistry 273:6402-6409 (1998), the disclosure of which is incorporated herein by reference in its entirety.

In some embodiments, upon administration of the composition to the patient, expression of CASQ2 is increased in a cardiac cell (e.g., a cardiac myocyte) in the patient. Expression of CASQ2 may be assessed, for example, by measuring CASQ2 protein or CASQ2 mRNA using gene expression assays known in the art and described herein. Upon administration of the composition to the patient, CASQ2 expression in the cardiac cell may be increased, for example, by from about 1.1-fold to about 10-fold, or more (e.g., about 1.1-fold, 1.2-fold, 1.3-fold, 1.4-fold, 1.5-fold, 1.6-fold, 1.7-fold, 1.8-fold, 1.9-fold, 2-fold, 2.1-fold, 2.2-fold, 2.3-fold, 2.4-fold, 2.5-fold, 2.6-fold, 2.7-fold, 2.8-fold, 2.9-fold, 3-fold, 3.1-fold, 3.2-fold, 3.3-fold, 3.4-fold, 3.5-fold, 3.6-fold, 3.7-fold, 3.8-fold, 3.9-fold, 4-fold, 4.1-fold, 4.2-fold, 4.3-fold, 4.4-fold, 4.5-fold, 4.6-fold, 4.7-fold, 4.8-fold, 4.9-fold, 5-fold, 5.1-fold, 5.2-fold, 5.3-fold, 5.4-fold, 5.5-fold, 5.6-fold, 5.7-fold, 5.8-fold, 5.9-fold, 6-fold, 6.1-fold, 6.2-fold, 6.3-fold, 6.4-fold, 6.5-fold, 6.6-fold, 6.7-fold, 6.8-fold, 6.9-fold, 7-fold, 7.1-fold, 7.2-fold, 7.3-fold, 7.4-fold, 7.5-fold, 7.6-fold, 7.7-fold, 7.8-fold, 7.9-fold, 8-fold, 8.1-fold, 8.2-fold, 8.3-fold, 8.4-fold, 8.5-fold, 8.6-fold, 8.7-fold, 8.8-fold, 8.9-fold, 9-fold, 9.1-fold, 9.2-fold, 9.3-fold, 9.4-fold, 9.5-fold, 9.6-fold, 9.7-fold, 9.8-fold, 9.8-fold, 10-fold, or more). For example, CASQ2 expression may be increased by from about 1.5-fold to about 3.5-fold in the cardiac cell (e.g., about .5-fold, 1.6-fold, 1.7-fold, 1.8-fold, 1.9-fold, 2-fold, 2.1-fold, 2.2-fold, 2.3-fold, 2.4-fold, 2.5-fold, 2.6-fold, 2.7-fold, 2.8-fold, 2.9-fold, 3-fold, 3.1-fold, 3.2-fold, 3.3-fold, 3.4-fold, or 3.5-fold). In some embodiments, CASQ2 expression is increased by from about 2-fold to about 3-fold in the cell (e.g., about 2-fold, 2.1-fold, 2.2-fold, 2.3-fold, 2.4-fold, 2.5-fold, 2.6-fold, 2.7-fold, 2.8-fold, 2.9-fold, or 3-fold). In some embodiments, CASQ2 expression is increased by from about 2.5-fold to about 3-fold in the cell (e.g., 2.5-fold, 2.6-fold, 2.7-fold, 2.8-fold, 2.9-fold, or 3-fold).

In some embodiments, upon administration of the composition to the patient, expression of RYR2, triadin, and/or junctin is not substantially altered in a cardiac cell (e.g., a cardiac myocyte) in the patient, as assessed, for example, by evaluating protein and/or mRNA expression. For example, upon administration of the composition to the patient, expression of RYR2, triadin, and/or junctin is increased and/or decreased by less than 10% (e.g., 9.9%, 9.8%, 9.7%, 9.6%, 9.5%, 9.4%, 9.3%, 9.2%, 9.1%, 9%, 8.9%, 8.8%, 8.7%, 8.6%, 8.5%, 8.4%, 8.3%, 8.2%, 8.1%, 8%, 7.9%, 7.8%, 7.7%, 7.6%, 7.5%, 7.4%, 7.3%, 7.2%, 7.1%, 7%, 6.9%, 6.8%, 6.7%, 6.6%, 6.5%, 6.4%, 6.3%, 6.2%, 6.1%, 6%, 5.9%, 5.8%, 5.7%, 5.6%, 5.5%, 5.4%, 5.3%, 5.2%, 5.1%, 5%, 4.9%, 4.8%, 4.7%, 4.6%, 4.5%, 4.4%, 4.3%, 4.2%, 4.1%, 4%, 3.9%, 3.8%, 3.7%, 3.6%, 3.5%, 3.4%, 3.3%, 3.2%, 3.1%, 3%, 2.9%, 2.8%, 2.7%, 2.6%, 2.5%, 2.4%, 2.3%, 2.2%, 2.1%, 2%, 1.9%, 1.8%, 1.7%, 1.6%, 1.5%, 1.4%, 1.3%, 1.2%, 1.1%, 1%, 0.9%, 0.8%, 0.7%, 0.6%, 0.5%, 0.4%, 0.3%, 0.2%, 0.1%, 0.01%, 0.001%, or less).

In some embodiments, the composition is administered to the patient by way of intravenous, intrathecal, intramuscular, intradermal, transdermal, parenteral, intranasal, subcutaneous, percutaneous, intratracheal, intraperitoneal, intraarterial, intravascular, inhalation, perfusion, lavage, and/or oral administration.

In another aspect, the invention features a kit that includes a composition containing a transgene encoding CASQ2. The kit may further include a package insert that instructs a user of the kit to administer the composition to a patient (e.g., a human patient) to treat dominant CPVT or a symptom thereof in the patient in accordance with the method of any of the above aspects of the invention and/or any of the above embodiments thereof.

In some embodiments of the preceding aspect, the CASQ2 transgene encodes a protein having an amino acid sequence that is at least 85% identical to the amino acid sequence of SEQ ID NO: 1 (e.g., 85%, 86%, 87%, 88%, 89%, 90%, 91%, 92%, 93%, 94%, 95%, 96%, 97%, 98%, 99%, 99.9%, or 100% identical to the amino acid sequence of SEQ ID NO: 1).

In some embodiments of the preceding aspect, the CASQ2 transgene encodes has a nucleic acid sequence that is at least 85% identical to the nucleic acid sequence of SEQ ID NO: 2 (e.g., 85%, 86%, 87%, 88%, 89%, 90%, 91%, 92%, 93%, 94%, 95%, 96%, 97%, 98%, 99%, 99.9%, or 100% identical to the nucleic acid sequence of SEQ ID NO: 2).

In some embodiments of the preceding aspect, the composition containing the CASQ2 transgene is a vector, such as a viral vector. The viral vector may be, for example, an adeno-associated virus (AAV), adenovirus, lentivirus, retrovirus, poxvirus, baculovirus, herpes simplex virus, or a vaccinia virus. In some embodiments, the viral vector is an AAV vector, such as AAV1, AAV2, AAV3, AAV4, AAV5, AAV6, AAV7, AAV8, AAV9, or AAVrh74 serotype vector. The AAV vector may be pseudotyped. In some embodiments, the pseudotyped AAV vector is AAV2/8 or AV2/9.

In some embodiments of the preceding aspect, the composition is a liposome, vesicle, synthetic vesicle, exosome, synthetic exosome, dendrimer, or nanoparticle.

In some embodiments of the preceding aspect, the transgene encoding CASQ2 is operably linked to a promoter that induces expression of the CASQ2 in a cardiac myocyte. For example, the promoter may be a desmin promoter, myosin light chain-2 promoter, alpha actin promoter, troponin 1 promoter, $Na^+/Ca^{2+}$ exchanger promoter, dystrophin promoter, creatine kinase promoter, alpha7 integrin promoter, brain natriuretic peptide promoter, alpha B-crystallin/small heat shock protein promoter, alpha myosin heavy chain promoter, or atrial natriuretic factor promoter.

## Definitions

As used herein, the term "about" refers to a value that is within 10% above or below the value being described.

As used herein, the terms "calmodulin 1" ("CALM1") and "calmodulin 3" ("CALM3") refer to the calmodulin calcium-binding protein expressed as secondary messengers, for example, in muscle cells (e.g., cardiac myocytes). CALM1 and CALM3 assist in regulating the cytosolic concentration of divalent calcium ions. An exemplary amino acid sequence of wild-type CALM1 is found in NCBI Reference Sequence No. NP_008819.1. Similarly, an exemplary amino acid sequence of wild-type CALM3 is found in NCBI Reference Sequence No. NP_001316851.1.

As used herein, the terms "calsequestrin 2" and "CASQ2" refer to the calcium-binding protein responsible for the regulating cytosolic concentrations of divalent calcium, for example, in muscle tissue, such as in cardiac myocytes. In humans, the naturally-occurring gene encoding CASQ2 is located on chromosome 1 and is also known as "PDIB2". The terms "calsequestrin 2," "CASQ2," and "PDIB2" are used interchangeably herein and refer not only to wild-type forms of CASQ2, but also to variants of wild-type CASQ2 proteins and nucleic acids encoding the same. The amino acid sequences of wild-type human CASQ2 is provided herein as SEQ ID NO: 1, and is characterized in the art as NCBI Reference Sequence No. NP_001223.2. Further, the nucleic acid sequence of wild-type human CASQ2 is provided herein as SEQ ID NO: 2, and is characterized in the art as NCBI Reference Sequence No. NM_001232.3. CASQ2 transgenes that may be used in conjunction with the compositions and methods described herein include those that encode a protein having an amino acid sequence that is at least 85% identical to the amino acid sequence of SEQ ID NO: 1 (e.g., 85%, 86%, 87%, 88%, 89%, 90%, 91%, 92%, 93%, 94%, 95%, 96%, 97%, 98%, 99%, 99.9%, or 100% identical to the amino acid sequence of SEQ ID NO: 1), as well as transgenes that encode a protein having one or more conservative amino acid substitutions (e.g., up to 5, up to 10, up to 15, up to 20, up to 25, up to 30, up to 35, up to 40, up to 45, or up to 50 conservative amino acid substitutions) and/or one or more nonconservative amino acid substitutions (e.g., up to 5, up to 10, up to 15, up to 20, up to 25, up to 30, up to 35, up to 40, up to 45, or up to 50 nonconservative amino acid substitutions) relative to the amino acid sequence of SEQ ID NO: 1. Exemplary CASQ2 transgenes that may be used in conjunction with the compositions and methods described herein include those that have a nucleic acid sequence that is at least 85% identical to the amino acid sequence of SEQ ID NO: 2 (e.g., 85%, 86%, 87%, 88%, 89%, 90%, 91%, 92%, 93%, 94%, 95%, 96%, 97%, 98%, 99%, 99.9%, or 100% identical to the amino acid sequence of SEQ ID NO: 2). CASQ2 transgenes herein further include those that encode fragments of the foregoing proteins that retain the ability to polymerize and bind divalent calcium, for example, with an affinity that is within up to 25% (e.g., with an affinity that is within 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, or 25%) of the affinity of wild-type CASQ2 and/or with a stoichiometry that deviates from that of wild-type CASQ2 by no more than 25% (e.g., no more than 25%, 24%, 23%, 22%, 21%, 20%, 19%, 18%, 17%, 16%, 15%, 14%, 13%, 12%, 11%, 10%, 9%, 8%, 7%, 6%, 5%, 4%, 3%, 2%, 1%, or less). Examples of assays that can be used to assess calcium binding affinity and stoichiometry are known in the art and described, for example, in Johnson et al. Methods Mol. Biol. 173:89-102 (2002), the disclosure of which is incorporated herein by reference in its entirety.

As used herein, the terms "catecholaminergic polymorphic ventricular tachycardia" and "CPVT" refer to an inherited channelopathy characterized by a high susceptibility of the patient to life threatening arrhythmias. Two forms of the disease have been described: autosomal dominant and autosomal recessive variants. The autosomal dominant form of the disease is associated with mutations in the cardiac ryanodine receptor type 2 (RYR2) gene, while the autosomal recessive variant is associated with mutations in the CASQ2 gene (see, for example, Priori et al., Circulation 104 (Supplement II):335 (2001), and Lahat et al., Am. J. Hum. Genet. 69:1378-1384 (2001), the disclosures of each of which are incorporated herein by reference in their entirety). Patients having the dominantly-inherited form of the disease, referred to interchangeably herein as "dominant CPVT" and "autosomal dominant CPVT," typically present with bidirectional and polymorphic ventricular tachycardia in response to sympathetic activation. The resting electrocardiography profiles of patients having dominant CPVT are unremarkable and heart structure is preserved. Arrhythmias in CPVT are elicited by calcium release events that are not triggered by an action potential, referred to herein as "spontaneous calcium releases" or "SCRs." SCR begins as a localized event involving a single calcium-release unit (CRU), but can also diffuse to neighboring CRUs, thus triggering more calcium release, resulting in a cell-wide calcium wave. When abnormal calcium release from the sarcoplasmic reticulum occurs, the ensuing action potential may propagate to the entire heart, resulting in an extrasystolic beat. When this chain of events becomes repetitive, triggered arrhythmic activity is elicited. Dominant CPVT is often caused by mutations in ryanodine receptor 2 (RYR2), which have been shown to facilitate the occurrence of SCRs during β adrenergic stimulation. The arrhythmias generated as a result of this dysregulation of calcium activity can lead to life-threatening arrhythmias (see, for example, Liu et al., Circulation Research 99:292-298 (2006), the disclosure of which is incorporated herein by reference in its entirety). Mutations in RYR2 that are associated with dominant CPVT include R4497C, N4104K, N4895D, R176Q, G178A, R420W, L433P, P1067S, S2246L, G2273R, F2307L, E2311D, L2344P, R2474S, T2504M, K3717R, L3778F, G3926D, G3946S, Q4159P, V4319-K4324dup, R4651I, V4771I, F4851L, A4860G, I4867M, P4902S, and R4959Q.

As used herein, mutations in proteins are represented using the form "AA1-N-AA2," wherein AA1 represents the amino acid occurring at position N of the wild-type form of the protein of interest, and AA2 represents the amino acid occurring at position N of the mutated form of the protein. For example, the RYR2 mutant "R4497C" refers to a variant form of RYR2 in which the arginine residue at position 4497 of the wild-type RYR2 protein is replaced with a cysteine residue. The RYR2 mutant "V4319-K4324dup" refers to a variant form of the RYR2 gene in which the nucleotides encoding amino acid residues V4319 through K4324 of wild-type RYR2 are duplicated, resulting in an insertion of an additional instance of the 18 nucleotides encoding these amino acids (e.g., an insertion of the residues GTCGAAGGTGCTAAAAAG (SEQ ID NO: 7) between nucleotides 12955 and 12956 of a wild-type RYR2 gene).

As used herein, the terms "conservative mutation," "conservative substitution," or "conservative amino acid substitution" refer to a substitution of one or more amino acids for one or more different amino acids that exhibit similar physicochemical properties, such as polarity, electrostatic charge, and steric volume. These properties are summarized for each of the twenty naturally-occurring amino acids in Table 1 below.

Table 1. Representative physicochemical properties of naturally-occurring amino acids

| Amino Acid | 3 Letter Code | 1 Letter Code | Side-chain Polarity | Electrostatic character at physiological pH (7.4) | Steric Volume† |
|---|---|---|---|---|---|
| Alanine | Ala | A | nonpolar | neutral | small |
| Arginine | Arg | R | polar | cationic | large |
| Asparagine | Asn | N | polar | neutral | intermediate |
| Aspartic acid | Asp | D | polar | anionic | intermediate |
| Cysteine | Cys | C | nonpolar | neutral | intermediate |
| Glutamic acid | Glu | E | polar | anionic | intermediate |
| Glutamine | Gln | Q | polar | neutral | intermediate |
| Glycine | Gly | G | nonpolar | neutral | small |
| Histidine | His | H | polar | Both neutral and cationic forms in equilibrium at pH 7.4 | large |
| Isoleucine | Ile | I | nonpolar | neutral | large |
| Leucine | Leu | L | nonpolar | neutral | large |
| Lysine | Lys | K | polar | cationic | large |
| Methionine | Met | M | nonpolar | neutral | large |
| Phenylalanine | Phe | F | nonpolar | neutral | large |
| Proline | Pro | P | nonpolar | neutral | intermediate |
| Serine | Ser | S | polar | neutral | small |
| Threonine | Thr | T | polar | neutral | intermediate |
| Tryptophan | Trp | W | nonpolar | neutral | bulky |
| Tyrosine | Tyr | Y | polar | neutral | large |
| Valine | Val | V | nonpolar | neutral | intermediate |
| †based on volume in $A^3$: 50-100 is small, 100-150 is intermediate, 150-200 is large, and >200 is bulky | | | | | |

From this table it is appreciated that the conservative amino acid families include, e.g., (i) G, A, V, L, I, P, and M; (ii) D and E; (iii) C, S and T; (iv) H, K and R; (v) N and Q; and (vi) F, Y and W. A conservative mutation or substitution is therefore one that substitutes one amino acid for a member of the same amino acid family (e.g., a substitution of Ser for Thr or Lys for Arg).

As used herein, the term "desmin promoter" refers to (i) a polynucleotide having the nucleic acid sequence of the endogenous human desmin promoter, defined by nucleotides -984 to +76, inclusive, with respect to the human desmin transcription start site, as well as to (ii) variants and functional portions thereof. For example, desmin promoters that may be used in conjunction with the compositions and methods of the disclosure include those that are at least 85% identical (e.g., 85%, 86% e.g., 85%, 86%, 87%, 88%, 89%, 90%, 91%, 92%, 93%, 94%, 95%, 96%, 97%, 98%, 99%, or 99.9% identical, or more) to the nucleic acid sequence of nucleotides -984 to +76, inclusive, with respect to the human desmin transcription start site and that promote the expression of a CASQ2 transgene in a cell (e.g., a eukaryotic cell, such as a mammalian cell, human cell, or human cardiomyocyte) when the transgene is operably linked to the promoter. Additional examples of desmin promoters that may be used in conjunction with the compositions and methods described herein are those described in Gao et al., Journal of Biological Chemistry 273:6402-6409 (1998), the disclosure of which is incorporated herein by reference in its entirety. As used herein in the context of a transcription regulatory element, such as a promoter, the term "functional portion" refers to a portion of a larger nucleic acid that retains the ability to stimulate transcription of a gene of interest in a target cell. For example, the endogenous human desmin promoter contains nucleotides -984 to +76, inclusive, with respect to the desmin transcription start site. Examples of functional portions of this locus that are able to retain the transcription-activating properties of the larger nucleic acid are regions containing nucleotides -973 to -693, inclusive, with respect to the desmin transcription start site. Additional examples are regions containing nucleotides -269 to +76, inclusive, with respect to the desmin transcription start site.

Similarly, as used herein, the terms "cytomegalovirus promoter," "myosin light chain-2 promoter," "alpha actin promoter," "troponin 1 promoter," "$Na^+/Ca^{2+}$ exchanger promoter," "dystrophin promoter," "creatine kinase promoter," "alpha7 integrin promoter," "brain natriuretic peptide promoter," "alpha B-crystallin/small heat shock protein promoter," "alpha myosin heavy chain promoter," and "atrial natriuretic factor promoter" refer to the wild-type forms of the endogenous human cytomegalovirus promoter, myosin light chain-2 promoter, alpha actin promoter, troponin 1 promoter, $Na^+/Ca^{2+}$ exchanger promoter, dystrophin promoter, creatine kinase promoter, alpha7 integrin promoter, brain natriuretic peptide promoter, alpha B-crystallin/small heat shock protein promoter, alpha myosin heavy chain promoter, and atrial natriuretic factor promoter, respectively, as well as to variants and functional portions thereof.

As used herein in the context of a mutation, the term "heterozygous" refers to an instance in which a patient has one mutant allele of a particular gene: either a mutant maternal allele or a mutant paternal allele.

As used herein in the context of a mutation, the term "homozygous" refers to an instance in which a patient has two mutant alleles of a particular gene: a mutant maternal allele and a mutant paternal allele.

As used herein, the term "gain-of-function mutation" refers to a mutation in which the function of a protein is augmented by virtue of the substitution, insertion, and/or deletion of one or more amino acids within the protein. For example, the ryanodine receptor 2 (RYR2) protein is a channel protein that mediates release of divalent calcium ions from the sarcoplasmic reticulum of cardiac myocytes into the cytosol. Exemplary gain-of-function mutations in the context of RYR2 are those that result in elevated RYR2-mediated calcium export from the sarcoplasmic reticulum. Gain-of-function mutations in RYR2 include R4497C, N4104K, N4895D, R176Q, G178A, R420W, L433P, P1067S, S2246L, G2273R, F2307L, E2311D, L2344P, R2474S, T2504M, K3717R, L3778F, G3926D, G3946S, Q4159P, V4319-K4324dup, R4651I, V4771I, F4851L, A4860G, I4867M, P4902S, and/or R4959Q, among others.

As used herein, the term "operably linked" refers to a first molecule (e.g., a first nucleic acid) joined to a second molecule (e.g., a second nucleic acid), wherein the molecules are so arranged that the first molecule affects the function of the second molecule. The two molecules may or may not be part of a single contiguous molecule and may or may not be adjacent to one another. For example, a promoter is operably linked to a transcribable polynucleotide molecule if the promoter modulates transcription of the transcribable polynucleotide molecule of interest in a cell. Additionally, two portions of a transcription regulatory element are operably linked to one another if they are joined such that the transcription-activating functionality of one portion is not adversely affected by the presence of the other portion. Two transcription regulatory elements may be operably linked to one another by way of a linker nucleic acid (e.g., an intervening non-coding nucleic acid) or may be operably linked to one another with no intervening nucleotides present.

"Percent (%) sequence identity" with respect to a reference polynucleotide or polypeptide sequence is defined as the percentage of nucleic acids or amino acids in a candidate sequence that are identical to the nucleic acids or amino acids in the reference polynucleotide or polypeptide sequence, after aligning the sequences and introducing gaps, if necessary, to achieve the maximum percent sequence identity. Alignment for purposes of determining percent nucleic acid or amino acid sequence identity can be achieved in various ways that are within the capabilities of one of skill in the art, for example, using publicly available computer software such as BLAST, BLAST-2, or Megalign software. Those skilled in the art can determine appropriate parameters for aligning sequences, including any algorithms needed to achieve maximal alignment over the full length of the sequences being compared. For example, percent sequence identity values may be generated using the sequence comparison computer program BLAST. As an illustration, the percent sequence identity of a given nucleic acid or amino acid sequence, A, to, with, or against a given nucleic acid or amino acid sequence, B, (which can alternatively be phrased as a given nucleic acid or amino acid sequence, A that has a certain percent sequence identity to, with, or against a given nucleic acid or amino acid sequence, B) is calculated as follows:

$$100 \text{ multiplied by (the fraction } X/Y)$$

where X is the number of nucleotides or amino acids scored as identical matches by a sequence alignment program (e.g., BLAST) in that program's alignment of A and B, and where Y is the total number of nucleic acids in B. It will be appreciated that where the length of nucleic acid or amino acid sequence A is not equal to the length of nucleic acid or amino acid sequence B, the percent sequence identity of A to B will not equal the percent sequence identity of B to A.

As used herein, the term "pharmaceutical composition" refers to a mixture containing a therapeutic agent, optionally in combination with one or more pharmaceutically acceptable excipients, diluents, and/or carriers, to be administered to a subject, such as a mammal, e.g., a human, in order to prevent, treat or control a particular disease or condition affecting or that may affect the subject.

As used herein, the term "pharmaceutically acceptable" refers to those compounds, materials, compositions, and/or dosage forms, which are suitable for contact with the tissues of a subject, such as a mammal (e.g., a human) without excessive toxicity, irritation, allergic response and other problem complications commensurate with a reasonable benefit/risk ratio.

As used herein, the terms "ryanodine receptor 2" and "RYR2" refer to the calcium channel protein located on the exterior of the sarcoplasmic reticulum, for example, in cardiac myocytes. RYR2 mediates the release of divalent calcium from the sarcoplasmic reticulum, which in turn stimulates action potentials that lead to heart contractions. An exemplary amino acid sequence of wild-type human RYR2 is found in NCBI Reference Sequence No. NP_001026.2.

As used herein, the terms "ryanodine receptor 2 agonist" and "RYR2 agonist" refer to agents that stimulate the calcium-release properties of the RYR2 channel protein. Exemplary RYR2 agonists include, without limitation, caffeine, epinephrine, trifluoroperazine, and maurocalcine, among others.

As used herein, the term "sample" refers to a specimen (e.g., blood, blood component (e.g., serum or plasma), urine, saliva, amniotic fluid, cerebrospinal fluid, tissue (e.g., placental or dermal), pancreatic fluid, chorionic villus sample, or cells) isolated from a subject. The subject may be, for example, a patient suffering from a disease described herein, such as a heritable cardiac disorder (e.g., a tachycardia, such as dominant catecholaminergic polymorphic ventricular tachycardia).

As used herein, the phrases "specifically binds" and "binds" refer to a binding reaction which is determinative of the presence of a particular molecule or ion, such as a divalent calcium ion, in a heterogeneous population of ions, salts, small molecules, and/or proteins that is recognized, e.g., by a ligand or receptor, such as a calcium-binding protein, with particularity. A ligand (e.g., a calcium-binding protein) that specifically binds to a species (e.g., a divalent ion) may bind to the species, e.g., with a $K_D$ of less than 1 mM. For example, a ligand that specifically binds to a species may bind to the species with a $K_D$ of up to 100 $\mu$M (e.g., between 1 pM and 100 $\mu$M). A ligand that does not exhibit specific binding to another molecule or ion may exhibit a $K_D$ of greater than 1 mM (e.g., 1 $\mu$M, 100 $\mu$M, 500 $\mu$M, 1 mM, or greater) for that particular molecule or ion. A variety of assay formats may be used to determine the affinity of a ligand for a specific protein. For example, solid-phase ELISA assays are routinely used to identify ligands that specifically bind a target protein. See, e.g., Harlow & Lane, Antibodies, A Laboratory Manual, Cold Spring Harbor Press, New York (1988) and Harlow & Lane, Using Antibodies, A Laboratory Manual, Cold Spring Harbor Press, New York (1999), for a description of assay formats and conditions that can be used to determine specific protein binding. Exemplary assays that can be used to determine the affinity of a ligand for a target ion (e.g., a divalent calcium ion) are described, e.g., in Johnson et al. Methods Mol. Biol. 173:89-102 (2002), the disclosure of which is incorporated herein by reference in its entirety.

As used herein, the terms "subject" and "patient" refer to an organism that receives treatment for a particular disease or condition as described herein (such as a heritable cardiac disorder, e.g., autosomal dominant catecholaminergic polymorphic ventricular tachycardia). Examples of subjects and patients include mammals, such as humans, receiving treatment for a disease or condition described herein.

As used herein, the term "tachycardia" refers to a heart rate exhibited by a patient (e.g., a human patient having dominant CPVT) that exceeds the normal resting heart rate for a given species. In the case of treatment of a human patient, tachycardia includes instances in which the patient's heart rate exceeds 100 beats per minute. Methods for monitoring tachycardia are known in the art and described, for example, in Example 1, below.

As used herein, the term "transcription regulatory element" refers to a nucleic acid that controls, at least in part, the transcription of a gene of interest. Transcription regulatory elements may include promoters, enhancers, and other nucleic acids (e.g., polyadenylation signals) that control or help to control gene transcription. Examples of transcription regulatory elements are described, for example, in Goeddel, Gene Expression Technology: Methods in Enzymology 185 (Academic Press, San Diego, CA, 1990).

As used herein, the terms "treat" or "treatment" refer to therapeutic treatment, in which the object is to prevent or slow down (lessen) an undesired physiological change or disorder, such as the progression of a heritable cardiac disorder, such as CPVT, and particularly, dominant CPVT. In the context of CPVT treatment, beneficial or desired clinical results include, but are not limited to, alleviation of symptoms, diminishment of extent of disease, stabilized (i.e., not worsening) state of disease, delay or slowing of disease progression, amelioration or palliation of the disease state, and remission (whether partial or total), whether detectable or undetectable. Treatment of a patient having CPVT (e.g., dominant CPVT) may manifest in one or more detectable changes, such as a decrease in the quantity or frequency of arrhythmia events (e.g., tachycardia events) exhibited by the patient in a specified time period following administration of a therapeutic agent described herein (e.g., a decrease in the quantity or frequency of arrhythmia events (e.g., tachycardia events) exhibited by the patient following administration of a vector, such as a viral vector, encoding a CASQ2 transgene described herein). For example, indications of successful treatment of a dominant CPVT patient using the compositions and methods described herein include a finding that the quantity and/or frequency of arrhythmia events (e.g., tachycardia events) exhibited by the patient has decreased, e.g., by 1%, 2%, 3%, 4%, 5%, 6%, 7%, 8%, 9%, 10%, 15%, 20%, 25%, 30%, 35%, 40%, 45%, 50%, 55%, 60%, 65%, 70%, 75%, 80%, 85%, 90%, 95%, or 100%, in a specified time period following administration of the therapeutic agent relative to the quantity and/or frequency of arrhythmia events (e.g., tachycardia events) exhibited by the patient a time period (e.g., a time period of the same length) occurring prior to administration of the therapeutic agent. Additional clinical indications of successful treatment of a CPVT patient include a finding that the average diastolic concentration of cytosolic calcium ([$Ca^{2+}$]) in a cardiac cell (e.g., in a cardiomyocyte) of the patient has decreased, for example, by 1%, 2%, 3%, 4%, 5%, 6%, 7%, 8%, 9%, 10%, 15%, 20%, 25%, 30%, 35%, 40%, 45%, 50%, 55%, 60%, 65%, 70%, 75%, or more, following administration of a therapeutic agent to the patient. Yet another indication of successful treatment is a finding that expression of CASQ2 in the patient (e.g., in a cardiac cell of the patient, such as a cardiomyocyte) has increased following administration of a therapeutic agent. The CASQ2 expression may increase, for instance, by 1%, 2%, 3%, 4%, 5%, 6%, 7%, 8%, 9%, 10%, 15%, 20%, 25%, 30%, 35%, 40%, 45%, 50%, 55%, 60%, 65%, 70%, 75%, 80%, 85%, 90%, 95%, 100%, 200%, 300%, 400%, 500%, 600%, 700%, 800%, 900%, 1-fold, 2-fold, 3-fold, 4-fold, 5-fold, 6-fold, 7-fold, 8-fold, 9-fold, 10-fold, or more. The concentration of CASQ2 protein may be determined ex vivo, for example, using protein detection assays known in the art, including ELISA assays described herein. The concentration of CASQ2-encoding nucleic acids may be determined ex vivo, for example, using nucleic acid detection assays (e.g., RNA Seq assays) described herein.

As used herein, the term "vector" refers to a nucleic acid, e.g., DNA or RNA, that may function as a vehicle for the delivery of a gene of interest into a cell (e.g., a mammalian cell, such as a human cell), such as for purposes of replication and/or expression. Exemplary vectors useful in conjunction with the compositions and methods described herein are plasmids, DNA vectors, RNA vectors, virions, or other suitable replicon (e.g., viral vector). A variety of vectors have been developed for the delivery of polynucleotides encoding exogenous proteins into a prokaryotic or eukaryotic cell. Examples of such expression vectors are disclosed in, e.g., WO 1994/11026, the disclosure of which is incorporated herein by reference. Expression vectors described herein contain a polynucleotide sequence as well as, e.g., additional sequence elements used for the expression of proteins and/or the integration of these polynucleotide sequences into the genome of a mammalian cell. Certain vectors that can be used for the expression of transgenes described herein include plasmids that contain regulatory sequences, such as promoter and enhancer regions, which direct gene transcription. Other useful vectors for expression of transgenes contain polynucleotide sequences that enhance the rate of translation of these genes or improve the stability or nuclear export of the mRNA that results from gene transcription. These sequence elements include, e.g., 5' and 3' untranslated regions, an internal ribosomal entry site (IRES), and polyadenylation signal site in order to direct efficient transcription of the gene carried on the expression vector. The expression vectors described herein may also contain a polynucleotide encoding a marker for selection of cells that contain such a vector. Examples of a suitable marker include genes that encode resistance to antibiotics, such as ampicillin, chloramphenicol, kanamycin, or nourseothricin.

## Brief Description of the Figures

**FIG. 1** is a diagram of the pAAV2.1-CMV-CASQ2-IRES-eGP construct used in the murine CASQ2 gene therapy experiments described in Example 1, below. The vector contains, in the 5'-to-3' direction, a 5' AAV2 inverted terminal repeat (ITR), cytomegalovirus (CMV) promoter, simian virus 40 (SV40) intron, wild-type murine calsequestrin 2 (CASQ2) transgene, internal ribosomal entry site (IRES), enhanced green fluorescent protein (eGFP) transgene, woodchuck posttranscriptional regulatory element (WPRE), bovine growth hormone (BGH) polyadenylation sequence, and 3' AAV2 ITR. The pAAV2.1-CMV-CASQ2-IRES-eGFP construct used in Example 1, below, was packaged into an AAV9 capsid, thereby generating a recombinant AAV2/9 vector.

**FIG. 2** is a graph showing the effect of the pAAV2.1-CMV-CASQ2-IRES-eGFP vector on the percentage of arrhythmic events in R4496C/+ heterozygous knock in mice (Ryr2-Het), a model for autosomal dominant CPVT, upon exposure to epinephrine (2 mg/kg) and caffeine (120 mg/kg). The black bar (left) indicates that all untreated RyR2-Het mice (n=5) exhibited arrhythmias following epinephrine and caffeine administration. The results on the right of the graph demonstrate that none of the RyR2-Het mice infected with the pAAV2.1-CMV-CASQ2-IRES-eGFP vector (n=4) developed arrhythmias.

**FIG. 3** is a graph showing mRNA expression of CASQ2, cardiac RYR2, triadin, and junctin in myocardial tissue of wild-type mice and RYR2 R4496C/+ mice that were either untreated or treated with the pAAV2.1-CMV-CASQ2-IRES-eGFP vector. Results are reported as fold expression of mRNA normalized to mRNA expression in wild type mice (WT, white bars). Black bars show fold expression of mRNA transcripts in untreated RyR2 R4496C/+ heterozygous knock in mice (Ryr2-Het) versus wild type (WT); grey bars show fold expression of mRNA transcripts in RyR2 R4496C/+ heterozygous knock in mice treated with the pAAV2.1-CMV-CASQ2-IRES-eGFP vector (Ryr2-Het AAV9-CASQ2) versus wild type (WT).

**FIG. 4** is a diagram showing various components of another AAV vector found to exhibit an anti-arrhythmic effect in R4496C/+ heterozygous knock in mice. The vector contains a CASQ2 expression cassette flanked by 5' and 3' AAV2 ITRs. The expression cassette includes a transgene encoding wild-type CASQ2 operably linked to a desmin promoter.

**FIG. 5** is a graph showing the effect of a recombinant, pseudotyped AAV2/8 containing the vector shown in FIG. 4 on the percentage of arrhythmic events in R4496C/+ heterozygous knock in mice (Ryr2-Het) upon exposure to epinephrine (2 mg/kg) and caffeine (120 mg/kg). The black bar (left) indicates that about 44% (4 out of 9) of untreated RyR2-Het mice (n=9) exhibited arrhythmias following epinephrine and caffeine administration. The results on the right of the graph demonstrate that none of the RyR2-Het mice infected with the rAAV2/8 vector encoding CASQ2 (n=9) developed arrhythmias.

**Detailed Description**

Described herein are compositions and methods treating catecholaminergic polymorphic ventricular tachycardia (CPVT), and particularly autosomal dominant CPVT, in a patient, such as a human patient. Dominant CPVT is caused by mutations in one or more proteins that regulate the concentration of divalent calcium ions in cardiac myocytes. For example, dominant CPVT is associated with various mutations in the ryanodine receptor 2 (RYR2) protein, particularly gain-of-function mutations that upregulate RYR2-mediated release of divalent calcium ions from the sarcoplasmic reticulum. As described below, this leads to a cascade of signaling events and muscle contraction events that terminate in a heartbeat. Dominant CPVT is also associated with loss-of-function mutations in calmodulin proteins, such as calmodulin 1 (CALM1) and CALM3. These proteins assist in regulating cytosolic calcium by binding and sequestering divalent calcium ions, thereby preventing the propagation of an action potential. Excess calcium release from the sarcoplasmic reticulum during the diastole - a period of cardiac relaxation - engenders an extrasystolic heartbeat, which can trigger an arrhythmia.

The present disclosure is based, in part, on the discovery that dominant CPVT can be treated without the need to provide the patient with a functional RYR2, CALM1, or CALM3 protein or a transgene encoding the same. Using the compositions and methods described herein, a patient, such as a human patient, having dominant CPVT may be treated by administration of a transgene encoding a functional calsequestrin 2 (CASQ2) protein. Administration of the CASQ2, in turn, restores calcium homeostasis in the heart, leading to a reduction in arrhythmia events and the return of a normal heartbeat.

Using the compositions and methods described herein, a transgene encoding a functional CASQ2 protein may be delivered to a patient by way of viral gene therapy, among other approaches described below, such as the use of a liposome, vesicle, exosome, dendrimer, or nanoparticle. The sections that follow provide a summary of the pathophysiology that underlies dominant CPVT, as well as a description of exemplary vectors and other gene delivery vehicles that may be used for to provide patients with a functional CASQ2 transgene.

**Dominant CPVT**

CPVT is an inherited channelopathy characterized by high susceptibility to life threatening arrhythmias. Two forms of the disease have been described: the autosomal dominant and the autosomal recessive variants. The first is associated with mutations in the cardiac RYR2 gene, while the autosomal recessive variant is associated with mutations in the cardiac CASQ2 gene (Priori et al., Circulation 104 (Supplement II):335 (2001), and Lahat et al., Am. J. Hum. Genet. 69:1378-1384 (2001)). Clinical observations have shown that patients having the dominant form of CPVT develop bidirectional and polymorphic ventricular tachycardia in response to sympathetic activation, whereas their resting electrocardiography profiles are unremarkable, and heart structure is preserved.

The pathology underlying dominant CPVT is linked to an abnormal function of the physiologic mechanism referred to as calcium-induced calcium release (CICR), which is fundamental for maintaining excitation-contraction coupling in the heart. The highly coordinated opening and closing of voltage-dependent ion channels located in the membrane of cardiac myocytes generates a cardiac action potential. During the plateau phase of the action potential, opening of voltage-dependent L-type $Ca^{2+}$ channels allows the influx of $Ca^{2+}$ in the plasmalemma. This process triggers the calcium transient and induces opening of sarcoplasmic reticulum $Ca^{2+}$ release channel: RYR2 (Bers, Nature 415:198-205 (2002)). These local releases occur at specialized structures referred to as calcium-release units (CRUs). The CRUs are preferentially localized at the level of the transverse tubules (T-tubules), where the membrane of the sarcoplasmic reticulum is juxtaposed to the cellular membrane. One CRU is formed by clusters of RYR2, spanning the sarcoplasmic reticulum membrane, that are in close proximity to the L-type $Ca^{2+}$ channels on the cell membrane (Franzini-Armstrong, et l., Ann. NY Acad. Sci. 1047:76-85 (2005)). The $Ca^{2+}$ released from the sarcoplasmic reticulum binds to troponin C and induces a series of allosteric changes in myosin filaments leading to muscle fiber contraction. The subsequent removal of $Ca^{2+}$ is mediated by the concomitant closing of the RYR2 and the action of sarcoplasmic reticulum $Ca^{2+}$ ATPase that pumps $Ca^{2+}$ back into the sarcoplasmic reticulum stores.

Another component of $Ca^{2+}$-transient termination is the $Na^{+}$-$Ca^{2+}$ exchanger (NCX). The NCX extrudes one $Ca^{2+}$ ion (two positive charges) for every three $Na^{+}$ ions (three positive charges) taken into the cell. Thus, the NCX removes $Ca^{2+}$ by generating a net inward depolarizing current: the transient inward current (Iti) (Pieske, et al., Circ. Res. 85:38-46 (1999)). The NCX becomes very important for the removal of $Ca^{2+}$ in conditions characterized by calcium overload, for example, in case of RYR2 genetic mutations.

Arrhythmias in CPVT are elicited by $Ca^{2+}$ release events that are not triggered by an action potential and are, therefore, referred to as spontaneous calcium releases (SCRs). SCR begins as a localized event involving a single CRU, but can also diffuse to neighboring CRUs triggering more $Ca^{2+}$ release to produce a cell-wide calcium wave. The probability that SCR will lead to a calcium wave is influenced by the balance between sarcoplasmic reticulum $Ca^{2+}$ content and the concentration of $Ca^{2+}$ that induces $Ca^{2+}$ release, the so-called sarcoplasmic reticulum calcium threshold. RYR2 function has a pivotal role in controlling this threshold. Several RYR2 mutations that are associated with CPVT have been suggested to act by decreasing the sarcoplasmic reticulum calcium threshold so that SCR is readily induced (Venetucci et al., Nat. Rev. Cardiol. 9:561-75 (2012)).

When abnormal $Ca^{2+}$ leakage occurs, cytosolic $Ca^{2+}$ concentration increases, and the cell must activate mechanisms to prevent disruption of $Ca^{2+}$ homeostasis and re-establish the physiological diastolic level of $Ca^{2+}$. $I_{ti}$ current produces transient membrane depolarizations after completion of the action potential, known as a delayed afterdepolarization (DAD). When the amplitude of a DAD reaches the voltage threshold for $Na^+$ channel activation, a triggered action potential is generated. Propagation of an action potential to the entire heart generates an extrasystolic beat. When this chain of events becomes repetitive and several DADs reach the threshold for the generation of propagating action potentials, triggered arrhythmic activity is elicited. In several studies, mutations of RYR2 have been shown to facilitate the occurrence of SCRs during β-adrenergic stimulation and, in turn, elicit DADs and triggered activity leading to life-threatening arrhythmias (Liu et al., Circulation Research 99:292-298 (2006)).

The generation and characterization of a RYR2 $^{R4496C/+}$ knock-in mouse model for autosomal dominant CPVT (see, for example, Cerrone et al., M, Circulation Research 96:e77-e82 (2005), and US Patent No. 7,741,529) has provided great insight into the pathomechanism of this disease. RyR2 R4496C/+ heterozygous mice recapitulate human CPVT and develop adrenergically-induced bidirectional and polymorphic ventricular arrhythmias. The R4496C mutation in mice, which corresponds to the R4497C mutation in humans, increases the sensitivity of the RYR2 channel to luminal calcium, thus facilitating the spontaneous release of calcium from the sarcoplasmic reticulum.

Exemplary mutations that may give rise to dominant CPVT are R4497C, N4104K, N4895D, R176Q, G178A, R420W, L433P, P1067S, S2246L, G2273R, F2307L, E2311D, L2344P, R2474S, T2504M, K3717R, L3778F, G3926D, G3946S, Q4159P, V4319-K4324dup, R4651I, V4771I, F4851L, A4860G, I4867M, P4902S, and R4959Q. Mutations in CALM1 that may lead to dominant CPVT include N97S and N53I, and an exemplary mutation in CALM3 that may lead to dominant CPVT is A103V.

It has presently been discovered that CPVT caused, e.g., by RYR2, CALM1, and/or CALM3 mutations that lead to arrhythmia, such as R4997C in humans, can be treated without the need to provide the patient with functional transgenes encoding these proteins. Rather, using the compositions and methods described herein, patients having dominant CPVT can be treated by administration of a delivery vehicle containing a CASQ2 transgene. Exemplary vehicles for the delivery of CASQ2 useful in conjunction with the compositions and methods described herein are described in detail below.

**CPVT Treatment by CASQ2 Gene Therapy**

*CASQ2 Transgenes*

Using the compositions and methods described herein, a transgene encoding CASQ2 can be provided to a patient, such as a human patient having dominant CPVT, so as to treat the pathology. The CASQ2 transgene may be delivered to the patient by way of a variety of vectors (e.g., viral vectors), among other compositions for gene therapy described herein. In some embodiments (e.g., in which a human patient is treated), the CASQ2 transgene encodes a protein having an amino acid sequence that is at least 85% identical (e.g., 85%, 86%, 87%, 88%, 89%, 90%, 91%, 92%, 93%, 94%, 95%, 96%, 97%, 98%, 99%, 99.9%, or 100% identical) to the amino acid sequence of wild-type human CASQ2, which is provided in SEQ ID NO: 1, shown below. CASQ2 transgenes for use in conjunction with the compositions and methods described herein include those that encode a protein having one or more conservative amino acid substitutions relative to the amino acid sequence of SEQ ID NO: 1. For example, the CASQ2 transgene may encode a protein having up to 50 conservative amino acid substitutions (e.g., 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50 conservative amino acid substitutions) relative to the amino acid sequence of SEQ ID NO: 1. The CASQ2 transgene may, additionally or alternatively, have one or more nonconservative amino acid substitutions relative to the amino acid sequence of SEQ ID NO: 1. For example, the CASQ2 transgene may encode a protein having up to 50 nonconservative amino acid substitutions (e.g., 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50 nonconservative amino acid substitutions) relative to the amino acid sequence of SEQ ID NO: 1.

In some embodiments, (e.g., in which a human patient is treated), the CASQ2 transgene has a nucleic acid sequence that is at least 85% identical (e.g., 85%, 86%, 87%, 88%, 89%, 90%, 91%, 92%, 93%, 94%, 95%, 96%, 97%, 98%, 99%, 99.9%, or 100% identical) to the nucleic acid sequence of wild-type human CASQ2, which is provided in SEQ ID NO: 2, shown below.

Exemplary wild-type CASQ2 amino acid and nucleic acid sequences useful in conjunction with the compositions and methods described herein are described in Table 2, below.

Table 2. Exemplary CASQ2 amino acid and nucleic acid sequences

| SEQ ID NO. | Description | Amino Acid/Nucleic Acid Sequence |
|---|---|---|
| 1 | Wild-type Human CASQ2 Protein | MKRTHLFIVGIYFLSSCRAEEGLNFPTYDGK DRVVSLSEKNFKQVLKKYDLLCLYYHEPVS SDKVTQKQFQLKEIVLELVAQVLEHKAIGFV MVDAKKEAKLAKKLGFDEEGSLYILKGDRTI EFDGEFAADVLVEFLLDLIEDPVEIISSKLEV QAFERIEDYIKLIGFFKSEDSEYYKAFEEAAE HFQPYIKFFATFDKGVAKKLSLKMNEVDFYE PFMDEPIAIPNKPYTEEELVEFVKEHQRPTL RRLRPEEMFETWEDDLNGIHIVAFAEKSDP DGYEFLEILKQVARDNTDNPDLSILWIDPDD FPLLVAYWEKTFKIDLFRPQIGVVNVTDADS VWMEIPDDDDLPTAEELEDWIEDVLSGKINT EDDDEDDDDDDNSDEEDNDDSDDDDDE |

(continued)

| SEQ ID NO. | Description | Amino Acid/Nucleic Acid Sequence |
|---|---|---|
| 2 | Wild-type Human CASQ2 cDNA (Excluding UTR Sequences) | ATGAAGAGAACTCACTTGTTTATTGTGGG GATTTATTTTCTGTCCTCTTGCAGGGCAGA AGAGGGGCTTAATTTCCCCACATATGATG GGAAGGACCGAGTGGTAAGTCTTTCCGA GAAGAACTTCAAGCAGGTTTTAAAGAAATA TGACTTGCTTTGCCTCTACTACCATGAGC CGGTGTCTTCAGATAAGGTCACGCAAAAA CAGTTCCAACTGAAAGAAATCGTGCTTGA GCTTGTGGCCCAGGTCCTTGAACATAAAG CTATAGGCTTTGTGATGGTGGATGCCAAG AAAGAAGCCAAGCTTGCCAAGAAACTGGG TTTTGATGAAGAAGGAAGCCTGTATATTCT TAAGGGTGATCGCACAATAGAGTTTGATG GCGAGTTTGCAGCTGATGTCTTGGTGGAG TTCCTCTTGGATCTAATTGAAGACCCAGT GGAGATCATCAGCAGCAAACTGGAAGTCC AAGCCTTCGAACGCATTGAAGACTACATC AAACTCATTGGCTTTTTCAAGAGTGAGGA CTCAGAATACTACAAGGCTTTTGAAGAAG CAGCTGAACACTTCCAGCCTTACATCAAA TTCTTTGCCACCTTTGACAAGGGGTTGC AAAGAAATTATCTTTGAAGATGAATGAGGT TGACTTCTATGAGCCATTTATGGATGAGC |

(continued)

| SEQ ID NO. | Description | Amino Acid/Nucleic Acid Sequence |
|---|---|---|
| | | CCATTGCCATCCCCAACAAACCTTACACA GAAGAGGAGCTGGTGGAGTTTGTGAAGG AACACCAAAGACCCACTCTACGTCGCCTG CGCCCAGAAGAAATGTTTGAAACATGGGA AGATGATTTGAATGGGATCCACATTGTGG CCTTTGCAGAGAAGAGTGATCCAGATGGC TACGAATTCCTGGAGATCCTGAAACAGGT TGCCCGGGACAATACTGACAACCCCGATC TGAGCATCCTGTGGATCGACCCGGACGA CTTTCCTCTGCTCGTTGCCTACTGGGAGA AGACTTTCAAGATTGACCTATTCAGGCCA CAGATTGGGGTGGTGAATGTCACAGATGC TGACAGTGTCTGGATGGAGATTCCAGATG ATGACGATCTTCCAACTGCTGAGGAGCTG GAGGACTGGATTGAGGATGTGCTTTCTGG AAAGATAAACACTGAAGATGATGATGAAG ATGATGATGATGATGATAATTCTGATGAAG AGGATAATGATGACAGTGATGACGATGAT GATGAATAG |
| 3 | Wild-type Murine CASQ2 Protein | MKRIYLLMVGVYLLSLSGAEEGLNFPTYDGK DRVVSLSEKNLKQMLKRYDLLCLYYHEPVS SDKVSQKQFQLKEIVLELVAQVLEHKNIGFV MVDSRKEAKLAKRLGFSEEGSLYVLKGDRT IEFDGEFAADVLVEFLLDLIEDPVEIVNNKLE VQAFERIEDQTKLLGFFKNEDSEYYKAFQEA AEHFQPYIKFFATFDKAVAKKLSLKMNEVGF YEPFMDEPNVIPNKPYTEEELVEFVKEHQR PTLRRLRPEDMFETWEDDLNGIHIVAFAEK SDPDGYEFLEILKQVARDNTDNPDLSILWID PDDFPLLVAYWEKTFKIDLFKPQIGVVNVTD ADSIWMEIPDDDDLPTAEELEDWIEDVLSGK INTEDDDNEDEDDDGDDNDDDDDDDDDND NSDEDNEDSDDDDDDDE |

(continued)

| SEQ ID NO. | Description | Amino Acid/Nucleic Acid Sequence |
|---|---|---|
| 4 | Wild-type Murine CASQ2 cDNA (Excluding UTR Sequences) | ATGAAGAGGATTTACC TGCTCATGGTGGGGGTTTATCTGCTGTCC CTGAGCGGGGCAGAAGAGGGGCTGAACT TCCCCACGTACGATGGGAAAGACCGAGT |

(continued)

| SEQ ID NO. | Description | Amino Acid/Nucleic Acid Sequence |
|---|---|---|
| | | GGTCAGCCTTTCTGAGAAGAACCTCAAGC AGATGTTGAAGAGATATGATTTGCTCTGTC TCTATTACCACGAACCTGTGTCTTCAGACA AGGTCTCACAAAAACAGTTCCAGCTGAAG GAGATTGTACTGGAGCTTGTGGCCCAGGT CCTGGAACATAAAAACATAGGCTTTGTGA TGGTGGATTCGAGGAAAGAGGCCAAGCTT GCTAAGAGGCTGGGATTCAGTGAAGAAG GAAGCCTGTATGTTCTGAAGGGTGACCGC ACGATTGAGTTTGACGGGGAGTTCGCAGC AGATGTCTTAGTGGAATTTCTCTTGGATCT CATTGAAGACCCAGTGGAGATCGTGAATA ACAAGCTGGAGGTCCAGGCCTTTGAGCG CATCGAGGACCAGACCAAGCTCCTTGGCT TCTTCAAGAATGAGGACTCAGAATATTACA AAGCATTCCAAGAGGCAGCTGAACACTTC CAGCCTTACATCAAGTTCTTTGCCACCTTT GACAAGGCGGTGGCAAAGAAGTTATCCTT GAAGATGAACGAAGTTGGCTTCTATGAGC CATTTATGGATGAGCCCAACGTCATCCCT AACAAACCGTACACAGAAGAGGAGCTTGT GGAGTTTGTGAAGGAACATCAAAGACCCA CCCTACGTCGCTTGCGCCCAGAGGACAT GTTTGAAACATGGGAAGACGACTTGAATG GGATCCACATCGTGGCCTTTGCGGAGAA GAGTGACCCAGATGGCTATGAGTTCCTAG AGATCCTGAAACAGGTTGCCCGGGACAAC ACTGACAATCCTGACTTGAGCATCTTGTG GATTGACCCAGATGACTTTCCACTGCTTG TTGCTTACTGGGAGAAGACTTTCAAGATT GACCTGTTCAAGCCACAGATTGGGGTGGT GAATGTAACCGATGCTGACAGTATCTGGA TGGAGATCCCAGATGATGACGACCTGCCC ACAGCTGAGGAGCTGGAAGACTGGATTG AAGATGTGCTTTCTGGAAAGATCAACACT GAAGATGATGACAATGAAGATGAAGATGA TGATGGTGATGACAACGATGACGATGATG |

(continued)

| SEQ ID NO. | Description | Amino Acid/Nucleic Acid Sequence |
|---|---|---|
| | | ATGACGACGATGATAATGATAATTCTGATG AGGATAATGAAGACAGTGATGATGACGAT GATGACGATGAATAG |

CASQ2 transgenes herein further include those that encode fragments of the foregoing proteins that retain the ability to polymerize and bind divalent calcium, for example, with an affinity that is within up to 25% (e.g., with an affinity that is within 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, or 25%) of the affinity of wild-type CASQ2 and/or with a stoichiometry that deviates from that of wild-type CASQ2 by no more than 25% (e.g., no more than 25%, 24%, 23%, 22%, 21%, 20%, 19%, 18%, 17%, 16%, 15%, 14%, 13%, 12%, 11%, 10%, 9%, 8%, 7%, 6%, 5%, 4%, 3%, 2%, 1%, or less). Examples of assays that can be used to assess calcium binding affinity and stoichiometry are known in the art and described, for example, in Johnson et al. Methods Mol. Biol. 173:89-102 (2002), the disclosure of which is incorporated herein by reference in its entirety.

*Transcription regulatory elements*

CASQ2 transgenes as described herein may be incorporated into a delivery vehicle, such as a vector, plasmid, or other nucleic acid delivery vehicle described herein, such that the transgene is operably linked to a promoter. Exemplary promoters useful in conjunction with the compositions and methods described herein are those that induce transgene expression in a cardiac cell in a patient (e.g., a human patient), such as a cardiac myocyte. For example, promoters that may be used to induce expression of CASQ2 in the gene delivery vehicles described herein include, without limitation, a desmin promoter, cytomegalovirus promoter, myosin light chain-2 promoter, alpha actin promoter, troponin 1 promoter, $Na^+/Ca^{2+}$ exchanger promoter, dystrophin promoter, creatine kinase promoter, alpha7 integrin promoter, brain natriuretic peptide promoter, alpha B-crystallin/small heat shock protein promoter, alpha myosin heavy chain promoter, or atrial natriuretic factor promoter.

**Vectors for Delivery of Exogenous Nucleic Acids to Target Cells**

*Viral vectors for nucleic acid delivery*

Viral genomes provide a rich source of vectors that can be used for the efficient delivery of a gene of interest into the genome of a target cell in a patient (e.g., a mammalian cell, such as a human cell). Viral genomes are particularly useful vectors for gene delivery because the polynucleotides contained within such genomes are typically incorporated into the genome of a target cell by generalized or specialized transduction. These processes occur as part of the natural viral replication cycle, and do not require added proteins or reagents in order to induce gene integration. Examples of viral vectors that may be used in conjunction with the compositions and methods described herein are AAV, retrovirus, adenovirus (e.g., Ad5, Ad26, Ad34, Ad35, and Ad48), parvovirus (e.g., adeno-associated viruses), coronavirus, negative strand RNA viruses such as orthomyxovirus (e.g., influenza virus), rhabdovirus (e.g., rabies and vesicular stomatitis virus), paramyxovirus (e.g. measles and Sendai), positive strand RNA viruses, such as picornavirus and alphavirus, and double stranded DNA viruses including adenovirus, herpesvirus (e.g., Herpes Simplex virus types 1 and 2, Epstein-Barr virus, cytomegalovirus), and poxvirus (e.g., vaccinia, modified vaccinia Ankara (MVA), fowlpox and canarypox). Other viruses that may be used in conjunction with the compositions and methods described herein include Norwalk virus, togavirus, flavivirus, reoviruses, papovavirus, hepadnavirus, and hepatitis virus, for example. Examples of retroviruses include: avian leukosis-sarcoma, mammalian C-type, B-type viruses, D-type viruses, HTLV-BLV group, lentivirus, spumavirus (Coffin, J. M., Retroviridae: The viruses and their replication, In Fundamental Virology, Third Edition, B. N. Fields, et al., Eds., Lippincott-Raven Publishers, Philadelphia, 1996). Other examples include murine leukemia viruses, murine sarcoma viruses, mouse mammary tumor virus, bovine leukemia virus, feline leukemia virus, feline sarcoma virus, avian leukemia virus, human T-cell leukemia virus, baboon endogenous virus, Gibbon ape leukemia virus, Mason Pfizer monkey virus, simian immunodeficiency virus, simian sarcoma virus, Rous sarcoma virus and lentiviruses. Other examples of vectors are described, for example, in US Patent No. 5,801,030, the disclosure of which is incorporated herein by reference as it pertains to viral vectors for use in gene therapy.

*AAV vectors for nucleic acid delivery*

In some embodiments, nucleic acids of the compositions and methods described herein are incorporated into rAAV vectors and/or virions in order to facilitate their introduction into a cell, such as a target cardiac cell (e.g., cardiac myocyte) in a patient. rAAV vectors useful in the conjunction with the compositions and methods described herein include recombinant nucleic acid constructs that contain (1) a CASQ2 transgene and (2) viral nucleic acids that facilitate integration and expression of the heterologous genes. The viral nucleic acids may include those sequences of AAV that are required in cis for replication and packaging (e.g., functional ITRs) of the DNA into a virion. Such rAAV vectors may also contain marker or reporter genes. Useful rAAV vectors include those having one or more of the naturally-occurring AAV genes deleted in whole or in part, but retain functional flanking ITR sequences. The AAV ITRs may be of any serotype (e.g., derived from serotype 2) suitable for a particular application. Methods for using rAAV vectors are described, for example, in Tai et al., J. Biomed. Sci. 7:279-291 (2000), and Monahan and Samulski, Gene Delivery 7:24-30 (2000), the disclosures of each of which are incorporated herein by reference as they pertain to AAV vectors for gene delivery.

The nucleic acids and vectors described herein can be incorporated into a rAAV virion in order to facilitate introduction of the nucleic acid or vector into a cell. The capsid proteins of AAV compose the exterior, non-nucleic acid portion of the virion and are encoded by the AAV cap gene. The cap gene encodes three viral coat proteins, VP1, VP2 and VP3, which are required for virion assembly. The construction of rAAV virions has been described, for example, in US Patent Nos. 5,173,414; 5,139,941; 5,863,541; 5,869,305; 6,057,152; and 6,376,237; as well as in Rabinowitz et al., J. Virol. 76:791-801 (2002) and Bowles et al., J. Virol. 77:423-432 (2003), the disclosures of each of which are incorporated herein by reference as they pertain to AAV vectors for gene delivery.

rAAV virions useful in conjunction with the compositions and methods described herein include those derived from a variety of AAV serotypes including AAV 1, 2, 3, 4, 5, 6, 7, 8 and 9. Construction and use of AAV vectors and AAV proteins of different serotypes are described, for example, in Chao et al., Mol. Ther. 2:619-623 (2000); Davidson et al., Proc. Natl. Acad. Sci. USA 97:3428-3432 (2000); Xiao et al., J. Virol. 72:2224-2232 (1998); Halbert et al., J. Virol. 74:1524-1532 (2000); Halbert et al., J. Virol. 75:6615-6624 (2001); and Auricchio et al., Hum. Molec. Genet. 10:3075-3081 (2001), the disclosures of each of which are incorporated herein by reference as they pertain to AAV vectors for gene delivery.

Also useful in conjunction with the compositions and methods described herein are pseudotyped rAAV vectors. Pseudotyped vectors include AAV vectors of a given serotype (e.g., AAV2) pseudotyped with a capsid gene derived from a serotype other than the given serotype (e.g., AAV1, AAV3, AAV4, AAV5, AAV6, AAV7, AAV8, or AAV9, among others). For example, a representative pseudotyped vector is an AAV2 vector encoding a therapeutic protein pseudotyped with a capsid gene derived from AAV serotype 8 or AAV serotype 9. Techniques involving the construction and use of pseudotyped rAAV virions are known in the art and are described, for example, in Duan et al., J. Virol. 75:7662-7671 (2001); Halbert et al., J. Virol. 74:1524-1532 (2000); Zolotukhin et al., Methods, 28:158-167 (2002); and Auricchio et al., Hum. Molec. Genet., 10:3075-3081 (2001).

AAV virions that have mutations within the virion capsid may be used to infect particular cell types more effectively than non-mutated capsid virions. For example, suitable AAV mutants may have ligand insertion mutations for the facilitation of targeting AAV to specific cell types. The construction and characterization of AAV capsid mutants including insertion mutants, alanine screening mutants, and epitope tag mutants is described in Wu et al., J. Virol. 74:8635-45 (2000). Other rAAV virions that can be used in methods of the invention include those capsid hybrids that are generated by molecular breeding of viruses as well as by exon shuffling. See, e.g., Soong et al., Nat. Genet., 25:436-439 (2000) and Kolman and Stemmer, Nat. Biotechnol. 19:423-428 (2001).

**Additional Methods for the Delivery of Transgenes to Target Cells**

*Transfection techniques*

Techniques that can be used to introduce a CASQ2 transgene, such as a CASQ2 transgene operably linked to a transcription regulatory element described herein, into a target cell are known in the art. For example, electroporation can be used to permeabilize mammalian cells (e.g., human target cells) by the application of an electrostatic potential to the cell of interest. Mammalian cells, such as human cells, subjected to an external electric field in this manner are subsequently predisposed to the uptake of exogenous nucleic acids. Electroporation of mammalian cells is described in detail, e.g., in Chu et al., Nucleic Acids Research 15:1311 (1987), the disclosure of which is incorporated herein by reference. A similar technique, Nucleofection™, utilizes an applied electric field in order to stimulate the uptake of exogenous polynucleotides into the nucleus of a eukaryotic cell. Nucleofection™ and protocols useful for performing this technique are described in detail, e.g., in Distler et al., Experimental Dermatology 14:315 (2005), as well as in US 2010/0317114, the disclosures of each of which are incorporated herein by reference.

Additional techniques useful for the transfection of target cells include the squeeze-poration methodology. This technique induces the rapid mechanical deformation of cells in order to stimulate the uptake of exogenous DNA through membranous pores that form in response to the applied stress. This technology is advantageous in that a vector is not required for delivery of nucleic acids into a cell, such as a human target cell. Squeeze-poration is described in detail, e.g., in Sharei et al., Journal of Visualized Experiments 81 :e50980 (2013), the disclosure of which is incorporated herein by reference.

Lipofection represents another technique useful for transfection of target cells. This method involves the loading of nucleic acids into a liposome, which often presents cationic functional groups, such as quaternary or protonated amines, towards the liposome exterior. This promotes electrostatic interactions between the liposome and a cell due to the anionic nature of the cell membrane, which ultimately leads to uptake of the exogenous nucleic acids, for example, by direct fusion of the liposome with the cell membrane or by endocytosis of the complex. Lipofection is described in detail, for example, in US Patent No. 7,442,386, the disclosure of which is incorporated herein by reference. Similar techniques that exploit ionic interactions with the cell membrane to provoke the uptake of foreign nucleic acids include contacting a cell with a cationic polymer-nucleic acid complex. Exemplary cationic molecules that associate with polynucleotides so as to impart a positive charge favorable for interaction with the cell membrane are activated dendrimers (described, e.g., in Dennig, Topics in Current Chemistry 228:227 (2003), the disclosure of which is incorporated herein by reference) and diethylaminoethyl (DEAE)-dextran, the use of which as a transfection agent is described in detail, for example, in Gulick et al., Current Protocols in Molecular Biology 40:1:9.2:9.2.1 (1997), the disclosure of which is incorporated herein by reference. Magnetic beads are another tool that can be used to transfect target cells in a mild and efficient manner, as this methodology utilizes an applied magnetic field in order to direct the uptake of nucleic acids. This technology is described in detail, for example, in US 2010/0227406, the disclosure of which is incorporated herein by reference.

Another useful tool for inducing the uptake of exogenous nucleic acids by target cells is laserfection, a technique that involves exposing a cell to electromagnetic radiation of a particular wavelength in order to gently permeabilize the cells and allow polynucleotides to penetrate the cell membrane. This technique is described in detail, e.g., in Rhodes et al., Methods in Cell Biology 82:309 (2007), the disclosure of which is incorporated herein by reference.

Microvesicles represent another potential vehicle that can be used to modify the genome of a target cell according to the methods described herein. For example, microvesicles that have been induced by the co-overexpression of the glycoprotein VSV-G with, e.g., a genome-modifying protein, such as a nuclease, can be used to efficiently deliver proteins into a cell that subsequently catalyze the site-specific cleavage of an endogenous polynucleotide sequence so as to prepare the genome of the cell for the covalent incorporation of a polynucleotide of interest, such as a gene or regulatory sequence. The use of such vesicles, also referred to as Gesicles, for the genetic modification of eukaryotic cells is described in detail, e.g., in Quinn et al., Genetic Modification of Target Cells by Direct Delivery of Active Protein [abstract]. In: Methylation changes in early embryonic genes in cancer [abstract], in: Proceedings of the 18th Annual Meeting of the American Society of Gene and Cell Therapy; 2015 May 13, Abstract No. 122.

*Incorporation of target genes by gene editing techniques*

In addition to the above, a variety of tools have been developed that can be used for the incorporation of a gene of interest into a target cell, such as a human cell. One such method that can be used for incorporating polynucleotides encoding target genes into target cells involves the use of transposons. Transposons are polynucleotides that encode transposase enzymes and contain a polynucleotide sequence or gene of interest flanked by 5' and 3' excision sites. Once a transposon has been delivered into a cell, expression of the transposase gene commences and results in active enzymes that cleave the gene of interest from the transposon. This activity is mediated by the site-specific recognition of transposon excision sites by the transposase. In some instances, these excision sites may be terminal repeats or inverted terminal repeats. Once excised from the transposon, the gene of interest can be integrated into the genome of a mammalian cell by transposase-catalyzed cleavage of similar excision sites that exist within the nuclear genome of the cell. This allows the gene of interest to be inserted into the cleaved nuclear DNA at the complementary excision sites, and subsequent covalent ligation of the phosphodiester bonds that join the gene of interest to the DNA of the mammalian cell genome completes the incorporation process. In certain cases, the transposon may be a retrotransposon, such that the gene encoding the target gene is first transcribed to an RNA product and then reverse-transcribed to DNA before incorporation in the mammalian cell genome. Exemplary transposon systems are the piggybac transposon (described in detail in, e.g., WO 2010/085699) and the sleeping beauty transposon (described in detail in, e.g., US 2005/0112764), the disclosures of each of which are incorporated herein by reference as they pertain to transposons for use in gene delivery to a cell of interest.

Another tool for the integration of target genes into the genome of a target cell is the clustered regularly interspaced short palindromic repeats (CRISPR)/Cas system, a system that originally evolved as an adaptive defense mechanism in bacteria and archaea against viral infection. The CRISPR/Cas system includes palindromic repeat sequences within plasmid DNA and an associated Cas9 nuclease. This ensemble of DNA and protein directs site specific DNA cleavage of a target sequence by first incorporating foreign DNA into CRISPR loci. Polynucleotides containing these foreign sequences and the repeat-spacer elements of the CRISPR locus are in turn transcribed in a host cell to create a guide RNA, which can subsequently anneal to a target sequence and localize the Cas9 nuclease to this site. In this manner, highly site-specific cas9-mediated DNA cleavage can be engendered in a foreign polynucleotide because the interaction that brings cas9 within close proximity of the target DNA molecule is governed by RNA:DNA hybridization. As a result, one can design a CRISPR/Cas system to cleave any target DNA molecule of interest. This technique has been exploited in order to edit eukaryotic genomes (Hwang et al., Nature Biotechnology 31:227 (2013)) and can be used as an efficient means of site-specifically editing target cell genomes in order to cleave DNA prior to the incorporation of a gene encoding a target gene. The use of CRISPR/Cas to modulate gene expression has been described in, for example, US Patent No. 8,697,359, the disclosure of which is incorporated herein by reference as it pertains to the use of the CRISPR/Cas system for genome editing. Alternative methods for site-specifically cleaving genomic DNA prior to the incorporation of a gene of interest in a target cell include the use of zinc finger nucleases (ZFNs) and transcription activator-like effector nucleases (TALENs). Unlike the CRISPR/Cas system, these enzymes do not contain a guiding polynucleotide to localize to a specific target sequence. Target specificity is instead controlled by DNA binding domains within these enzymes. The use of ZFNs and TALENs in genome editing applications is described, e.g., in Urnov et al., Nature Reviews Genetics 11:636 (2010); and in Joung et al., Nature Reviews Molecular Cell Biology 14:49 (2013), the disclosure of each of which are incorporated herein by reference as they pertain to compositions and methods for genome editing.

Additional genome editing techniques that can be used to incorporate polynucleotides encoding target genes into the genome of a target cell include the use of ARCUS™ meganucleases that can be rationally designed so as to site-specifically cleave genomic DNA. The use of these enzymes for the incorporation of genes encoding target genes into the genome of a mammalian cell is advantageous in view of the defined structure-activity relationships that have been established for such enzymes. Single chain meganucleases can be modified at certain amino acid positions in order to create nucleases that selectively cleave DNA at desired locations, enabling the site-specific incorporation of a target gene into the nuclear DNA of a target cell. These single-chain nucleases have been described extensively in, for example, US Patent Nos. 8,021,867 and US 8,445,251, the disclosures of each of which are incorporated herein by reference as they pertain to compositions and methods for genome editing.

**Methods of Measuring Transgene Expression**

The expression level of a CASQ2 transgene expressed by a target cell in a patient (e.g., a cardiac myocyte) can be ascertained, for example, by evaluating the concentration or relative abundance of mRNA transcripts derived from transcription of the CASQ2 transgene. Additionally or alternatively, gene expression can be determined by evaluating the concentration or relative abundance of CASQ2 protein produced by transcription and translation of a CASQ2 transgene. Protein concentrations can also be assessed using functional assays, such as calcium abundance assays. The sections that follow describe exemplary techniques that can be used to measure the expression level of a CASQ2 transgene upon delivery to a patient, such as a patient having dominant CPVT as described herein. Transgene expression can be evaluated by a number of methodologies known in the art, including, but not limited to, nucleic acid sequencing, microarray analysis, proteomics, in-situ hybridization (e.g., fluorescence in-situ hybridization (FISH)), amplification-based assays, in situ hybridization, fluorescence activated cell sorting (FACS), northern analysis and/or PCR analysis of mRNAs.

*Nucleic acid detection*

Nucleic acid-based methods for determining transgene expression detection methods datasets suitable for analysis in conjunction with the compositions and methods described herein include imaging-based techniques (e.g., Northern blotting or Southern blotting), which may be used in conjunction with cells obtained from a patient following administration of the transgene. Northern blot analysis is a conventional technique well known in the art and is described, for example, in Molecular Cloning, a Laboratory Manual, second edition, 1989, Sambrook, Fritch, Maniatis, Cold Spring Harbor Press, 10 Skyline Drive, Plainview, NY 11803-2500. Typical protocols for evaluating the status of genes and gene products are found, for example in Ausubel et al., eds., 1995, Current Protocols In Molecular Biology, Units 2 (Northern Blotting), 4 (Southern Blotting), 15 (Immunoblotting) and 18 (PCR Analysis).

Transgene detection techniques that may be used in conjunction with the compositions and methods described herein to evaluate CASQ2 expression further include microarray sequencing experiments (e.g., Sanger sequencing and next-generation sequencing methods, also known as high-throughput sequencing or deep sequencing). Exemplary next generation sequencing technologies include, without limitation, Illumina sequencing, Ion Torrent sequencing, 454 sequencing, SOLiD sequencing, and nanopore sequencing platforms. Additional methods of sequencing known in the art can also be used. For instance, transgene expression at the mRNA level may be determined using RNA-Seq (e.g., as described in Mortazavi et al., Nat. Methods 5:621-628 (2008) the disclosure of which is incorporated herein by reference in their entirety). RNA-Seq is a robust technology for monitoring expression by direct sequencing the RNA molecules in a sample. Briefly, this methodology may involve fragmentation of RNA to an average length of 200 nucleotides, conversion to cDNA by random priming, and synthesis of double-stranded cDNA (e.g., using the Just cDNA DoubleStranded cDNA Synthesis Kit from Agilent Technology). Then, the cDNA is converted into a molecular library for sequencing by addition of sequence adapters for each library (e.g., from Illumina®/Solexa), and the resulting 50-100 nucleotide reads are mapped onto the genome.

Transgene expression levels may be determined using microarray-based platforms (e.g., single-nucleotide polymorphism arrays), as microarray technology offers high resolution. Details of various microarray methods can be found in the literature. See, for example, U.S. Pat. No. 6,232,068 and Pollack et al., Nat. Genet. 23:41-46 (1999), the disclosures of each of which are incorporated herein by reference in their entirety. Using nucleic acid microarrays, mRNA samples are reverse transcribed and labeled to generate cDNA. The probes can then hybridize to one or more complementary nucleic acids arrayed and immobilized on a solid support. The array can be configured, for example, such that the sequence and position of each member of the array is known. Hybridization of a labeled probe with a particular array member indicates that the sample from which the probe was derived expresses that gene. Expression level may be quantified according to the amount of signal detected from hybridized probe-sample complexes. A typical microarray experiment involves the following steps: 1) preparation of fluorescently labeled target from RNA isolated from the sample, 2) hybridization of the labeled target to the microarray, 3) washing, staining, and scanning of the array, 4) analysis of the scanned image and 5) generation of gene expression profiles. One example of a microarray processor is the Affymetrix GENECHIP® system, which is commercially available and comprises arrays fabricated by direct synthesis of oligonucleotides on a glass surface. Other systems may be used as known to one skilled in the art.

Amplification-based assays also can be used to measure the expression level of a transgene in a target cell following delivery to a patient. In such assays, the nucleic acid sequences of the gene act as a template in an amplification reaction (for example, PCR, such as qPCR). In a quantitative amplification, the amount of amplification product is proportional to the amount of template in the original sample. Comparison to appropriate controls provides a measure of the expression level of the gene, corresponding to the specific probe used, according to the principles described herein. Methods of real-time qPCR using TaqMan probes are well known in the art. Detailed protocols for real-time qPCR are provided, for example, in Gibson et al., Genome Res. 6:995-1001 (1996), and in Heid et al., Genome Res. 6:986-994 (1996), the disclosures of each of which are incorporated herein by reference in their entirety. Levels of gene expression as described herein can be determined by RT-PCR technology. Probes used for PCR may be labeled with a detectable marker, such as, for example, a radioisotope, fluorescent compound, bioluminescent compound, a chemiluminescent compound, metal chelator, or enzyme.

*Protein detection*

Transgene expression can additionally be determined by measuring the concentration or relative abundance of a corresponding protein product (e.g., CASQ2) encoded by a gene of interest. Protein levels can be assessed using standard detection techniques known in the art. Protein expression assays suitable for use with the compositions and methods described herein include proteomics approaches, immunohistochemical and/or western blot analysis, immunoprecipitation, molecular binding assays, ELISA, enzyme-linked immunofiltration assay (ELIFA), mass spectrometry, mass spectrometric immunoassay, and biochemical enzymatic activity assays. In particular, proteomics methods can be used to generate large-scale protein expression datasets in multiplex. Proteomics methods may utilize mass spectrometry to detect and quantify polypeptides (e.g., proteins) and/or peptide microarrays utilizing capture reagents (e.g., antibodies) specific to a panel of target proteins to identify and measure expression levels of proteins expressed in a sample (e.g., a single cell sample or a multi-cell population).

Exemplary peptide microarrays have a substrate-bound plurality of polypeptides, the binding of an oligonucleotide, a peptide, or a protein to each of the plurality of bound polypeptides being separately detectable. Alternatively, the peptide microarray may include a plurality of binders, including, but not limited to, monoclonal antibodies, polyclonal antibodies, phage display binders, yeast two-hybrid binders, aptamers, which can specifically detect the binding of specific oligonucleotides, peptides, or proteins. Examples of peptide arrays may be found in U.S. Patent Nos. 6,268,210, 5,766,960, and 5,143,854, the disclosures of each of which are incorporated herein by reference in their entirety.

Mass spectrometry (MS) may be used in conjunction with the methods described herein to identify and characterize transgene expression in a cell from a patient (e.g., a human patient) following delivery of the transgene. Any method of MS known in the art may be used to determine, detect, and/or measure a protein or peptide fragment of interest, e.g., LC-MS, ESI-MS, ESI-MS/MS, MALDI-TOF-MS, MALDI-TOF/TOF-MS, tandem MS, and the like. Mass spectrometers generally contain an ion source and optics, mass analyzer, and data processing electronics. Mass analyzers include scanning and ion-beam mass spectrometers, such as time-of-flight (TOF) and quadruple (Q), and trapping mass spectrometers, such as ion trap (IT), Orbitrap, and Fourier transform ion cyclotron resonance (FT-ICR), may be used in the methods described herein. Details of various MS methods can be found in the literature. See, for example, Yates et al., Annu. Rev. Biomed. Eng. 11:49-79, 2009, the disclosure of which is incorporated herein by reference in its entirety.

Prior to MS analysis, proteins in a sample obtained from the patient can be first digested into smaller peptides by chemical (e.g., via cyanogen bromide cleavage) or enzymatic (e.g., trypsin) digestion. Complex peptide samples also benefit from the use of front-end separation techniques, e.g., 2D-PAGE, HPLC, RPLC, and affinity chromatography. The digested, and optionally separated, sample is then ionized using an ion source to create charged molecules for further analysis. Ionization of the sample may be performed, e.g., by electrospray ionization (ESI), atmospheric pressure chemical ionization (APCI), photoionization, electron ionization, fast atom bombardment (FAB)/liquid secondary ionization (LSIMS), matrix assisted laser desorption/ionization (MALDI), field ionization, field desorption, thermospray/plasmaspray ionization, and particle beam ionization. Additional information relating to the choice of ionization method is known to those of skill in the art.

After ionization, digested peptides may then be fragmented to generate signature MS/MS spectra. Tandem MS, also known as MS/MS, may be particularly useful for analyzing complex mixtures. Tandem MS involves multiple steps of MS selection, with some form of ion fragmentation occurring in between the stages, which may be accomplished with individual mass spectrometer elements separated in space or using a single mass spectrometer with the MS steps separated in time. In spatially separated tandem MS, the elements are physically separated and distinct, with a physical connection between the elements to maintain high vacuum. In temporally separated tandem MS, separation is accomplished with ions trapped in the same place, with multiple separation steps taking place over time. Signature MS/MS spectra may then be compared against a peptide sequence database (e.g., SEQUEST). Post-translational modifications to peptides may also be determined, for example, by searching spectra against a database while allowing for specific peptide modifications.

**Pharmaceutical Compositions**

The CASQ2 transgenes described herein may be incorporated into a vehicle for administration into a patient, such as a human patient suffering from dominant CPVT, as described herein. Pharmaceutical compositions containing vectors, such as viral vectors, that contain the CASQ2 transgene can be prepared using methods known in the art. For example, such compositions can be prepared using, e.g., physiologically acceptable carriers, excipients or stabilizers (Remington's Pharmaceutical Sciences 16th edition, Osol, A. Ed. (1980); incorporated herein by reference), and in a desired form, e.g., in the form of lyophilized formulations or aqueous solutions.

Mixtures of the nucleic acids and viral vectors described herein may be prepared in water suitably mixed with one or more excipients, carriers, or diluents. Dispersions may also be prepared in glycerol, liquid polyethylene glycols, and mixtures thereof and in oils. Under ordinary conditions of storage and use, these preparations may contain a preservative to prevent the growth of microorganisms. The pharmaceutical forms suitable for injectable use include sterile aqueous solutions or dispersions and sterile powders for the extemporaneous preparation of sterile injectable solutions or dispersions (described in US 5,466,468, the disclosure of which is incorporated herein by reference). In any case the formulation may be sterile and may be fluid to the extent that easy syringability exists. Formulations may be stable under the conditions of manufacture and storage and may be preserved against the contaminating action of microorganisms, such as bacteria and fungi. The carrier can be a solvent or dispersion medium containing, for example, water, ethanol, polyol (e.g., glycerol, propylene glycol, and liquid polyethylene glycol, and the like), suitable mixtures thereof, and/or vegetable oils. Proper fluidity may be maintained, for example, by the use of a coating, such as lecithin, by the maintenance of the required particle size in the case of dispersion and by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminum monostearate and gelatin.

For example, a solution containing a pharmaceutical composition described herein may be suitably buffered, if necessary, and the liquid diluent first rendered isotonic with sufficient saline or glucose. These particular aqueous solutions are especially suitable for intravenous, intramuscular, subcutaneous, and intraperitoneal administration. In this connection, sterile aqueous media that can be employed will be known to those of skill in the art in light of the present disclosure. For example, one dosage may be dissolved in 1 ml of isotonic NaCl solution and either added to 1000 ml of hypodermoclysis fluid or injected at the proposed site of infusion. Some variation in dosage will necessarily occur depending on the condition of the subject being treated. The person responsible for administration will, in any event, determine the appropriate dose for the individual subject. Moreover, for human administration, preparations may meet sterility, pyrogenicity, general safety, and purity standards as required by FDA Office of Biologics standards.

**Routes of Administration and Dosing**

Viral vectors, such as AAV vectors and others described herein, containing the transgene may be administered to a patient (e.g., a human patient) by a variety of routes of administration. The route of administration may vary, for example, with the onset and severity of disease, and may include, e.g., intravenous, intrathecal, intradermal, transdermal, parenteral, intramuscular, intranasal, subcutaneous, percutaneous, intratracheal, intraperitoneal, intraarterial, intravascular, inhalation, perfusion, lavage, and oral administration. Intravascular administration includes delivery into the vasculature of a patient. In some embodiments, the administration is into a vessel considered to be a vein (intravenous), and in some administration, the administration is into a vessel considered to be an artery (intraarterial). Veins include, but are not limited to, the internal jugular vein, a peripheral vein, a coronary vein, a hepatic vein, the portal vein, great saphenous vein, the pulmonary vein, superior vena cava, inferior vena cava, a gastric vein, a splenic vein, inferior mesenteric vein, superior mesenteric vein, cephalic vein, and/or femoral vein. Arteries include, but are not limited to, coronary artery, pulmonary artery, brachial artery, internal carotid artery, aortic arch, femoral artery, peripheral artery, and/or ciliary artery. It is contemplated that delivery may be through or to an arteriole or capillary.

Treatment regimens may vary, and often depend on disease severity and the age, weight, and sex of the patient. Treatment may include administration of vectors (e.g., viral vectors) or other agents described herein as useful for the introduction of a transgene into a target cell in various unit doses. Each unit dose will ordinarily contain a predetermined-quantity of the therapeutic composition.

In cases in which the transgene is delivered to the patient by way of viral gene therapy (e.g., by way of administration of an AAV vector described herein, such as an AAV1, AAV2, AAV3, AAV4, AAV5, AAV6, AAV7, AAV8, AAV9, AAVrh74, AAV2/8, or AAV2/9, vector), the viral vector may be administered to the patient at a dose of, for example, from about $1 \times 10^6$ genome copies (GC)/kg to about $1 \times 10^{18}$ GC/kg (e.g., at a dose of about $1 \times 10^6$ GC/kg, $2 \times 10^6$ GC/kg, $3 \times 10^6$ GC/kg, $4 \times 10^6$ GC/kg, $5 \times 10^6$ GC/kg, $6 \times 10^6$ GC/kg, $7 \times 10^6$ GC/kg, $8 \times 10^6$ GC/kg, $9 \times 10^6$ GC/kg, $1 \times 10^7$ GC/kg, $2 \times 10^7$ GC/kg, $3 \times 10^7$ GC/kg, $4 \times 10^7$ GC/kg, $5 \times 10^7$ GC/kg, $6 \times 10^7$ GC/kg, $7 \times 10^7$ GC/kg, $8 \times 10^7$ GC/kg, $9 \times 10^7$ GC/kg, $1 \times 10^8$ GC/kg, $2 \times 10^8$ GC/kg, $3 \times 10^8$ GC/kg, $4 \times 10^8$ GC/kg, $5 \times 10^8$ GC/kg, $6 \times 10^8$ GC/kg, $7 \times 10^8$ GC/kg, $8 \times 10^8$ GC/kg, $9 \times 10^8$ GC/kg, $1 \times 10^9$ GC/kg, $2 \times 10^9$ GC/kg, $3 \times 10^9$ GC/kg, $4 \times 10^9$ GC/kg, $5 \times 10^9$ GC/kg, $6 \times 10^9$ GC/kg, $7 \times 10^9$ GC/kg, $8 \times 10^9$ GC/kg, $9 \times 10^9$ GC/kg, $1 \times 10^{10}$ GC/kg, $2 \times 10^{10}$ GC/kg, $3 \times 10^{10}$ GC/kg, $4 \times 10^{10}$ GC/kg, $5 \times 10^{10}$ GC/kg, $6 \times 10^{10}$ GC/kg, $7 \times 10^{10}$ GC/kg, $8 \times 10^{10}$ GC/kg, $9 \times 10^{10}$ GC/kg, $1 \times 10^{11}$ GC/kg, $2 \times 10^{11}$ GC/kg, $3 \times 10^{11}$ GC/kg, $4 \times 10^{11}$ GC/kg, $5 \times 10^{11}$ GC/kg, $6 \times 10^{11}$ GC/kg, $7 \times 10^{11}$ GC/kg, $8 \times 10^{11}$ GC/kg, $9 \times 10^{11}$ GC/kg, $1 \times 10^{12}$ GC/kg, $2 \times 10^{12}$ GC/kg, $3 \times 10^{12}$ GC/kg, $4 \times 10^{12}$ GC/kg, $5 \times 10^{12}$ GC/kg, $6 \times 10^{12}$ GC/kg, $7 \times 10^{12}$ GC/kg, $8 \times 10^{12}$ GC/kg, $9 \times 10^{12}$ GC/kg, $1 \times 10^{13}$ GC/kg, $2 \times 10^{13}$ GC/kg, $3 \times 10^{13}$ GC/kg, $4 \times 10^{13}$ GC/kg, $5 \times 10^{13}$ GC/kg, $6 \times 10^{13}$ GC/kg, $7 \times 10^{13}$ GC/kg, $8 \times 10^{13}$ GC/kg, $9 \times 10^{13}$ GC/kg, $1 \times 10^{14}$ GC/kg, $2 \times 10^{14}$ GC/kg, $3 \times 10^{14}$ GC/kg, $4 \times 10^{14}$ GC/kg, $5 \times 10^{14}$ GC/kg, $6 \times 10^{14}$ GC/kg, $7 \times 10^{14}$ GC/kg, $8 \times 10^{14}$ GC/kg, $9 \times 10^{14}$ GC/kg, $1 \times 10^{15}$ GC/kg, $2 \times 10^{15}$ GC/kg, $3 \times 10^{15}$ GC/kg, $4 \times 10^{15}$ GC/kg, $5 \times 10^{15}$ GC/kg, $6 \times 10^{15}$ GC/kg, $7 \times 10^{15}$ GC/kg, $8 \times 10^{15}$ GC/kg, $9 \times 10^{15}$ GC/kg, $1 \times 10^{16}$ GC/kg, $2 \times 10^{16}$ GC/kg, $3 \times 10^{16}$ GC/kg, $4 \times 10^{16}$ GC/kg, $5 \times 10^{16}$ GC/kg, $6 \times 10^{16}$ GC/kg, $7 \times 10^{16}$ GC/kg, $8 \times 10^{16}$ GC/kg, $9 \times 10^{16}$ GC/kg, $1 \times 10^{17}$ GC/kg, $2 \times 10^{17}$ GC/kg, $3 \times 10^{17}$ GC/kg, $4 \times 10^{17}$ GC/kg, $5 \times 10^{17}$ GC/kg, $6 \times 10^{17}$ GC/kg, $7 \times 10^{17}$ GC/kg, $8 \times 10^{17}$ GC/kg, $9 \times 10^{17}$ GC/kg, or $1 \times 10^{18}$ GC/kg). In some embodiments, the vector is administered to the patient at a dose of from about $1 \times 10^8$ GC/kg to about $1 \times 10^{16}$ GC/kg (e.g., at a dose of about $1 \times 10^8$ GC/kg, $2 \times 10^8$ GC/kg, $3 \times 10^8$ GC/kg, $4 \times 10^8$ GC/kg, $5 \times 10^8$ GC/kg, $6 \times 10^8$ GC/kg, $7 \times 10^8$ GC/kg, $8 \times 10^8$ GC/kg, $9 \times 10^8$ GC/kg, $1 \times 10^9$ GC/kg, $2 \times 10^9$ GC/kg, $3 \times 10^9$ GC/kg, $4 \times 10^9$ GC/kg, $5 \times 10^9$ GC/kg, $6 \times 10^9$ GC/kg, $7 \times 10^9$ GC/kg, $8 \times 10^9$ GC/kg, $9 \times 10^9$ GC/kg, $1 \times 10^{10}$ GC/kg, $2 \times 10^{10}$ GC/kg, $3 \times 10^{10}$ GC/kg, $4 \times 10^{10}$ GC/kg, $5 \times 10^{10}$ GC/kg, $6 \times 10^{10}$ GC/kg, $7 \times 10^{10}$ GC/kg, $8 \times 10^{10}$ GC/kg, $9 \times 10^{10}$ GC/kg, $1 \times 10^{11}$ GC/kg, $2 \times 10^{11}$ GC/kg, $3 \times 10^{11}$ GC/kg, $4 \times 10^{11}$ GC/kg, $5 \times 10^{11}$ GC/kg, $6 \times 10^{11}$ GC/kg, $7 \times 10^{11}$ GC/kg, $8 \times 10^{11}$ GC/kg, $9 \times 10^{11}$ GC/kg, $1 \times 10^{12}$ GC/kg, $2 \times 10^{12}$ GC/kg, $3 \times 10^{12}$ GC/kg, $4 \times 10^{12}$ GC/kg, $5 \times 10^{12}$ GC/kg, $6 \times 10^{12}$ GC/kg, $7 \times 10^{12}$ GC/kg, $8 \times 10^{12}$ GC/kg, $9 \times 10^{12}$ GC/kg, $1 \times 10^{13}$ GC/kg, $2 \times 10^{13}$ GC/kg, $3 \times 10^{13}$ GC/kg, $4 \times 10^{13}$ GC/kg, $5 \times 10^{13}$ GC/kg, $6 \times 10^{13}$ GC/kg, $7 \times 10^{13}$ GC/kg, $8 \times 10^{13}$ GC/kg, $9 \times 10^{13}$ GC/kg, $1 \times 10^{14}$ GC/kg, $2 \times 10^{14}$ GC/kg, $3 \times 10^{14}$ GC/kg, $4 \times 10^{14}$ GC/kg, $5 \times 10^{14}$ GC/kg, $6 \times 10^{14}$ GC/kg, $7 \times 10^{14}$ GC/kg, $8 \times 10^{14}$ GC/kg, $9 \times 10^{14}$ GC/kg, $1 \times 10^{15}$ GC/kg, $2 \times 10^{15}$ GC/kg, $3 \times 10^{15}$ GC/kg, $4 \times 10^{15}$ GC/kg, $5 \times 10^{15}$ GC/kg, $6 \times 10^{15}$ GC/kg, $7 \times 10^{15}$ GC/kg, $8 \times 10^{15}$ GC/kg, $9 \times 10^{15}$ GC/kg, or $1 \times 10^{16}$ GC/kg). In some embodiments, the vector is administered to the patient at a dose of from about $1 \times 10^{10}$ GC/kg to about $1 \times 10^{14}$ GC/kg (e.g., at a dose of about $1 \times 10^{10}$ GC/kg, $2 \times 10^{10}$ GC/kg, $3 \times 10^{10}$ GC/kg, $4 \times 10^{10}$ GC/kg, $5 \times 10^{10}$ GC/kg, $6 \times 10^{10}$ GC/kg, $7 \times 10^{10}$ GC/kg, $8 \times 10^{10}$ GC/kg, $9 \times 10^{10}$ GC/kg, $1 \times 10^{11}$ GC/kg, $2 \times 10^{11}$ GC/kg, $3 \times 10^{11}$ GC/kg, $4 \times 10^{11}$ GC/kg, $5 \times 10^{11}$ GC/kg, $6 \times 10^{11}$ GC/kg, $7 \times 10^{11}$ GC/kg, $8 \times 10^{11}$ GC/kg, $9 \times 10^{11}$ GC/kg, $1 \times 10^{12}$ GC/kg, $2 \times 10^{12}$ GC/kg, $3 \times 10^{12}$ GC/kg, $4 \times 10^{12}$ GC/kg, $5 \times 10^{12}$ GC/kg, $6 \times 10^{12}$ GC/kg, $7 \times 10^{12}$ GC/kg, $8 \times 10^{12}$ GC/kg, $9 \times 10^{12}$ GC/kg, $1 \times 10^{13}$ GC/kg, $2 \times 10^{13}$ GC/kg, $3 \times 10^{13}$ GC/kg, $4 \times 10^{13}$ GC/kg, $5 \times 10^{13}$ GC/kg, $6 \times 10^{13}$ GC/kg, $7 \times 10^{13}$ GC/kg, $8 \times 10^{13}$ GC/kg, $9 \times 10^{13}$ GC/kg, or $1 \times 10^{14}$ GC/kg).

## Examples

The following examples are put forth so as to provide those of ordinary skill in the art with a description of how the compositions and methods described herein may be used, made, and evaluated, and are intended to be purely exemplary of the invention and are not intended to limit the scope of what the inventors regard as their invention.

### Example 1. Delivery of CASQ2 to heterozygous RYR 4496C/+ mice eliminates arrhythmia in a manner independent of expression of RYR2, triadin, and junctin

### Materials and Methods

*Animal use*

Animals were maintained and bred at the Charles River Laboratories in Calco, Italy, and transferred to the ICS Maugeri Spa-SB for characterization of the phenotype. Animals were maintained and studied according to the protocols approved by the committee for the animals' well-being by the University of Pavia.

*Generation of knock-in R4496C RYR2 murine model*

The experiments described in this example utilized a heterozygous RYR2 $^{R4496C/+}$ knock-in mouse model of dominant CPVT, which recapitulates the phenotype of the human RYR2 R4997C mutation. Details regarding the production of this mouse model are provided in US Patent No. 7,741,529, the disclosure of which is incorporated herein by reference as it pertains to the generation of the RYR2 $^{R4496C/+}$ knock-in model.

*Vector design and production*

A detailed description of the design of one of the vectors employed in these experiments is provided in US Patent No. 8,859,517. Briefly, a pseudotyped adeno-associated serotype 9 vector (AAV2/9) was generated containing the coding sequence, without UTR sequences, of wild-type murine CASQ2 (NCBI Reference Sequence No. NM_009814.2). Wild-type murine CASQ2 was cloned into pGEM-T-Easy vector (Promega). By enzymatic digestion using EcoRI, the insert corresponding to the CASQ2 transgene was excised from the pGEM vector and subcloned in bis-cistronic pIRES vector (BD Biosciences, Cat. No: 631605, Clontech Palo Alto CA, USA). Subsequently, the fragment corresponding to the CASQ2 transgene followed by the IRES sequence was subcloned via PCR amplification using specific primers (Forward: 5'-CACAGCGGCCGCACAATGAAGAGGATTTACCTGCTCATGG-3' (SEQ ID NO: 5) and Reverse 5'-CGAAGCATTAACCCTCACTAAAGGG-3' (SEQ ID NO: 6) containing the NotI enzymatic site. The amplicon was inserted into the adenoviral backbone vector pAAV-2.1-eGFP, serotype 9 (containing a bovine growth hormone (BGH) polyA sequence and CMV promoter), provided by the Adeno-Associated Virus (AAV) vector Core facility (Tigem, Napoli, Italy), by way of enzymatic digestion with Not I. All plasmids were sequenced.

The AAV production was done in collaboration with the Tigem AAV Vector core facility.

The AAV vector was produced using a transient transfection of 3 plasmids in HEK 293 cells: pAd helper, pAAV rep-cap (packaging), pAAV Cis (including the CASQ2 insert, cloned in the pAAV2.1-CMV-eGFP plasmid MCS). Real time PCR and dot blot analysis was conducted to assess viral titer, and SDS PAGE followed by Coomassie staining was performed to evaluate the presence and purity of capsid proteins. Infectivity and sterility were also assessed. The service returned with a viral preparation in PBS with a total yield in excess of $1 \times 10^{12}$ genome copies. All AAV2/9-WT-mCASQ2-IRES-eGFP stocks were frozen at - 80°C in single aliquots and thawed during the surgical procedure.

Similar recombinant AAV production methods were used to furnish a second vector investigated in these experiments. The second vector was a pseudotyped AAV2/8 vector containing a transgene encoding wild-type CASQ2 operably linked to a desmin promoter. Various components of this second vector are shown in FIG. 4.

*Quantitative real time PCR*

Total RNA from cardiac tissue of RyR2 R4496C/+ mice and from RyR2 R4496C/+ heterozygous knock-in mice infected with the AAV9-WT-mCASQ2- IRES-eGFP viral vector was purified with RNeasy mini kit (Qiagen). A total amount of 1 μg template RNA per reaction was used for the RT-PCR assay performed with iScript cDNA Synthesis kit (Bio-Rad Laboratories, Inc., USA). Real Time quantification of mRNAs of various genes of interest (RyR2 CASQ2, Triadin, Junctin) and the reference gene (GAPDH) was performed in optical 96-well plates using CFX96 detection module (Bio-Rad Laboratories, Inc.). All samples were analyzed in triplicate with SsoFast EvaGreen Supermix using specific primer mix and 20 ng of cDNA template with primers reported previously (Denegri et al., Circulation 129:267381 (2014)). Real-time PCR was performed using the CFX96 Real-Time PCR Detection System and analyzed using the Bio-Rad CFX96 Manager Software (Version 1.5).

*In vivo experiments*

The AAV vector containing the construct shown in FIG. 1 was delivered via intraperitoneal injection of 100 μl of purified virus at a dose of $6.4 \times 10^{11}$ genome copies to n=4 neonatal mice with a 25 gauge syringe on the eighth postnatal day (P8). A total of n=5 littermates were not treated.

At 7 weeks of age, electrocardiography (ECG) and radiotelemetry monitors (Data Sciences International) were implanted subcutaneously under general anaesthesia (Isoflurane 1.5-3%) in all mice. After 72 hours of recovery from surgery, phenotype characterization was performed. Basal ECG was recorded for to assess for the presence of spontaneous arrhythmias. Immediately after, the susceptibility of mice to adrenergically-induced arrhythmias was tested by administering epinephrine (2 mg/kg) and caffeine (120 mg/kg) by intraperitoneal injection (Cerrone et al., M, Circulation Research 96:e77-e82 (2005), Liu et al., Circulation Research 99:292-298 (2006)). All animals were freely moving while ECG recordings were performed.

This experiment was then repeated using the AAV2/8 vector shown in FIG. 4 in order to investigate the anti-arrhythmic effects of this construct in a model organism for dominantly inherited CPVT. RyR2 R4496C/+ heterozygous mice (n=9) were administered the AAV2/8 vector at a dose of $6 \times 10^{13}$ vector genomes (vg) per kg. Non-treated RyR2 R4496C/+ heterozygous mice (n=9) were used as a control. At two months post administration, mice were evaluated by electrophysiological analysis in vivo (ECG) under epinephrine and caffeine stimulation.

**Results**

As shown in FIG. 2, administration of an AAV vector encoding the CASQ2 transgene to RYR2 $^{R4996C/+}$ mice resulted in a complete suppression of caffeine- and epinephrine-induced arrhythmia. Following β-adrenergic stimulation, none of the n=4 AAV CASQ2-treated mice exhibited arrhythmic activity, while all of the n=5 untreated mice demonstrated arrhythmia. Moreover, the therapeutic effects of CASQ2 delivery in RYR2 $^{R4996C/+}$ mice were not dependent upon the activity of RYR2 or the membrane-associated proteins, triadin and junctin. As shown in FIG. 3, administration of the AAV CASQ2 vector resulted in elevated CASQ2 expression in cardiac myocytes, but did not substantially alter RYR2, triadin, or junctin expression in such cells.

As shown in FIG. 5, similar results were obtained using the AAV2/8 construct. Particularly, administration of the AAV2/8-desmin-CASQ2 vector resulted in a complete suppression of caffeine- and epinephrine-induced arrhythmia in RYR2 $^{R4996C/+}$ mice, while 44% of the untreated mice demonstrated arrhythmia.

Collectively, these results demonstrate that CASQ2 transgene delivery in murine models of dominant CPVT fosters the correction of bidirectional and polymorphic arrhythmias. Surprisingly, CASQ2 gene transfer alone, without the need for RYR2 delivery, was found to prevent the physiological sinus rhythm that triggers arrhythmias due to adrenergic activation. CASQ2 gene therapy in murine dominant CPVT models resulted in augmented CASQ2 expression in cardiac myocytes and restored anti-arrhythmic cardiac contractile function.

In view of these results and the description provided herein, CASQ2 gene therapy can be used as a paradigm for the treatment of dominant CPVT, as illustrated in Example 2, below.

**Example 2. Treatment of dominant CPVT in a human patient by administration of a viral vector containing a CASQ2 transgene**

Using conventional molecular biology techniques known in the art, a gene encoding a CASQ2 protein, such as the wild-type human CASQ2 protein having the amino acid sequence of SEQ ID NO: 1 (or a variant thereof having at least 85% sequence identity to the amino acid sequence of SEQ ID NO: 1), can be incorporated into a viral vector and formulated for administration to a human patient. For example, a patient suffering from dominant CPVT, can be administered an AAV vector containing a CASQ2 transgene under the control of a transcriptional regulatory element that promotes CASQ2 expression in cardiac myocytes. The AAV vector may be a pseudotyped vector that incorporates the CASQ2 transgene between the 5' and 3' inverted terminal repeats of AAV2 and that is encapsidated by AAV8 or AAV9 capsid proteins (e.g., an AAV2/8 or AAV2/9 vector). The AAV vector can be administered to the subject by a variety of routes of administration, such as intravenously, intramuscularly, or subcutaneously, among others.

Following administration of the vector to a patient, a practitioner of skill in the art can monitor the expression of the CASQ2 gene, and the patient's improvement in response to the therapy, by a variety of methods. For example, a physician can monitor the patient's cardiac function by analyzing the quantity or frequency of arrhythmia events exhibited by the patient following administration of the transgene. A finding that the patient exhibits less or no arrhythmia activity in a specified time period following administration of the CASQ2 transgene relative to an equivalent time period assessed prior to administration of the transgene may indicate that the patient is responding favorably to the treatment. Subsequent doses can be determined and administered as needed.

### Other Embodiments

All publications, patents, and patent applications mentioned in this specification are incorporated herein by reference to the same extent as if each independent publication or patent application was specifically and individually indicated to be incorporated by reference.

While the invention has been described in connection with specific embodiments thereof, it will be understood that it is capable of further modifications and this application is intended to cover any variations, uses, or adaptations of the invention following, in general, the principles of the invention and including such departures from the invention that come within known or customary practice within the art to which the invention pertains and may be applied to the essential features hereinbefore set forth, and follows in the scope of the claims.

Other embodiments are within the claims.

## Claims

**1.** A method of treating dominant catecholaminergic polymorphic ventricular tachycardia (CPVT) in a human patient in need thereof, the method comprising administering to the patient a therapeutically effective amount of a composition comprising a transgene encoding calsequestrin 2 (CASQ2).

**2.** A method of reducing arrhythmia in a human patient diagnosed as having dominant CPVT, the method comprising administering to the patient a therapeutically effective amount of a composition comprising a transgene encoding CASQ2.

**3.** A method of restoring calcium homeostasis in a human patient diagnosed as having dominant CPVT, the method comprising administering to the patient a therapeutically effective amount of a composition comprising a transgene encoding CASQ2.

**4.** A method of reducing delayed afterdepolarization events in a human patient diagnosed as having dominant CPVT, the method comprising administering to the patient a therapeutically effective amount of a composition comprising a transgene encoding CASQ2.

**5.** A composition comprising a transgene encoding calsequestrin 2 (CASQ2), for use in the therapeutic treatment of dominant catecholaminergic polymorphic ventricular tachycardia (CPVT) in a human patient in need thereof.

**6.** A composition comprising a transgene encoding calsequestrin 2 (CASQ2), for use in reducing arrhythmia in a human patient diagnosed as having dominant CPVT.

**7.** A composition comprising a transgene encoding calsequestrin 2 (CASQ2), for use in restoring calcium homeostasis in a human patient diagnosed as having dominant CPVT.

**8.** A composition comprising a transgene encoding calsequestrin 2 (CASQ2), for use in reducing delayed afterdepolarization events in a human patient diagnosed as having dominant CPVT.

**9.** The method or the composition for use according to any of claims 1 to 8, wherein the patient exhibits a gain-of-function mutation in an endogenous gene encoding ryanodine receptor 2 (RYR2).

**10.** The method or the composition for use according to claim 9, wherein the mutation in RYR2 results in an increase in cytosolic calcium release in a cardiac myocyte that is contacted with a RYR2 agonist relative to a cardiac myocyte that is not contacted with the RYR2 agonist.

**11.** The method or the composition for use according to claim 9 or 10, wherein the mutation in RYR2 is R4497C, N4104K, N4895D, R176Q, G178A, R420W, L433P, P1067S, S2246L, G2273R, F2307L, E2311D, L2344P, R2474S, T2504M, K3717R, L3778F, G3926D, G3946S, Q4159P, V4319-K4324dup, R4651I, V4771I, F4851L, A4860G, I4867M, P4902S, and/or R4959Q.

**12.** The method or the composition for use according to any of claims 1 to 8, wherein the patient exhibits a mutation in an endogenous gene encoding a calmodulin.

**13.** The method or the composition for use according to claim 12, wherein the calmodulin is CALM1.

**14.** The method or the composition for use according to claim 13, wherein the mutation reduces CALM1 inhibition of calcium release from the sarcoplasmic reticulum of a cardiac myocyte.

**15.** The method or the composition for use according to claim 13, wherein the CALM1 harboring the mutation directly activates calcium release from the sarcoplasmic reticulum of a cardiac myocyte.

**16.** The method or the composition for use according to claim 13, wherein the mutation increases the stability of an open conformation of RYR2 in a CALM1-RYR2 complex present in an aqueous solution having a concentration of $Ca^{2+}$ of from about 50 nM to about 5 $\mu$M.

**17.** The method or the composition for use according to any of claims 13 to 16, wherein the mutation in CALM1 is N97S or N53I.

**18.** The method or the composition for use according to claim 12, wherein the calmodulin is CALM3.

**19.** The method or the composition for use according to claim 18, wherein the CALM3 harboring the mutation directly activates calcium release from the sarcoplasmic reticulum in a cardiac myocyte.

**20.** The method or the composition for use according to claim 18 or 19, wherein the CALM3 mutation is A103V.

**21.** The method or the composition for use according to any of claims 1 to 20, wherein the mutation is heterozygous.

**22.** The method or the composition for use according to any of claims 1-21, wherein the CASQ2 transgene encodes a protein having an amino acid sequence that is at least 85% identical to the amino acid sequence of SEQ ID NO: 1.

**23.** The method or the composition for use according to claim 22, wherein the CASQ2 transgene encodes a protein having an amino acid sequence that is at least 90% identical to the amino acid sequence of SEQ ID NO: 1.

**24.** The method or the composition for use according to claim 23, wherein the CASQ2 transgene encodes a protein having an amino acid sequence that is at least 95% identical to the amino acid sequence of SEQ ID NO: 1.

**25.** The method or the composition for use according to claim 24, wherein the CASQ2 transgene encodes a protein having the amino acid sequence of SEQ ID NO: 1.

**26.** The method or the composition for use according to any of claims 1 to 25, wherein the CASQ2 transgene has a nucleic acid sequence that is at least 85% identical to the nucleic acid sequence of SEQ ID NO: 2.

**27.** The method or the composition for use according to claim 26, wherein the CASQ2 transgene has a nucleic acid sequence that is at least 90% identical to the nucleic acid sequence of SEQ ID NO: 2.

**28.** The method or the composition for use according to claim 27, wherein the CASQ2 transgene has a nucleic acid sequence that is at least 95% identical to the nucleic acid sequence of SEQ ID NO: 2.

**29.** The method or the composition for use according to claim 28, wherein the CASQ2 transgene has the nucleic acid sequence of SEQ ID NO: 2.

**30.** The method or the composition for use according to any of claims 1 to 29, wherein the composition is a vector.

**31.** The method or the composition for use according to claim 30, wherein the vector is a viral vector.

**32.** The method or the composition for use according to claim 31, wherein the viral vector is selected from the group consisting of adeno-associated virus (AAV), adenovirus, lentivirus, retrovirus, poxvirus, baculovirus, herpes simplex virus, vaccinia virus, and a synthetic virus.

**33.** The method or the composition for use according to claim 32, wherein the synthetic virus is chimeric virus, mosaic virus, or pseudotyped virus, and/or comprises a foreign protein, synthetic polymer, nanoparticle, or small molecule.

**34.** The method or the composition for use according to claim 32, wherein the viral vector is an AAV.

**35.** The method or the composition for use according to claim 34, wherein the AAV is an AAV1, AAV2, AAV3, AAV4, AAV5, AAV6, AAV7, AAV8, AAV9, or AAVrh74 serotype.

**36.** The method or the composition for use according to claim 31, wherein the viral vector is a pseudotyped AAV.

**37.** The method or the composition for use according to claim 36, wherein the pseudotyped AAV is AAV2/8.

**38.** The method or the composition for use according to claim 36, wherein the pseudotyped AAV is AAV2/9.

**39.** The method or the composition for use according to claim 34, wherein the AAV comprises a recombinant capsid protein.

**40.** The method or the composition for use according to any of claims 1 to 29, wherein the composition is a liposome, vesicle, synthetic vesicle, exosome, synthetic exosome, dendrimer, or nanoparticle.

**41.** The method or the composition for use according to any of claims 1 to 40, wherein the transgene is operably linked to a promoter that induces expression of the CASQ2 in a cardiac myocyte.

**42.** The method or the composition for use according to claim 41, wherein the promoter is a desmin promoter, cytomegalovirus promoter, myosin light chain-2 promoter, alpha actin promoter, troponin 1 promoter, $Na^+/Ca^{2+}$ exchanger promoter, dystrophin promoter, creatine kinase promoter, alpha7 integrin promoter, brain natriuretic peptide promoter, alpha B-crystallin/small heat shock protein promoter, alpha myosin heavy chain promoter, or atrial natriuretic factor promoter.

**43.** The method or the composition for use according to claim 42, wherein the promoter is a desmin promoter.

**44.** The method or the composition for use according to any of claims 1 to 43, wherein upon administration of the composition to the patient, expression of CASQ2 is increased in a cardiac cell in the patient.

**45.** The method or the composition for use according to claim 44, wherein the cardiac cell is a cardiac myocyte.

**46.** The method or the composition for use according to claim 44 or 45, wherein the expression of CASQ2 is assessed by measuring CASQ2 protein in the cell.

**47.** The method or the composition for use according to claim 44 or 45, wherein expression of CASQ2 is assessed by measuring CASQ2 mRNA in the cell.

**48.** The method or the composition for use according to claim 47, wherein expression of CASQ2 mRNA is increased by from about 1.5-fold to about 3.5-fold in the cell.

**49.** The method or the composition for use according to claim 48, wherein expression of CASQ2 mRNA is increased by from about 2-fold to about 3-fold in the cell.

**50.** The method or the composition for use according to claim 49, wherein expression of CASQ2 mRNA is increased by from about 2.5-fold to about 3-fold in the cell.

**51.** The method or the composition for use according to any of claims 1 to 50, wherein upon administration of the composition to the patient, expression of RYR2, triadin, and/or junctin is not substantially altered in a cardiac cell in the patient.

**52.** The method or the composition for use according to any of claims 1 to 51, wherein the composition is administered to the patient by way of intravenous, intrathecal, intradermal, transdermal, parenteral, intramuscular, intranasal, subcutaneous, percutaneous, intratracheal, intraperitoneal, intraarterial, intravascular, inhalation, perfusion, lavage, and/or oral administration.

**53.** A kit comprising a composition comprising a transgene encoding CASQ2 and a package insert, wherein the package insert instructs a user of the kit to administer the composition to a patient suffering from dominant CPVT in accordance with the method according to any of claims 1 to 52.

**54.** The kit according to claim 53, wherein the composition is a vector.

**55.** The kit according to claim 54, wherein the vector is a viral vector.

**56.** The kit according to claim 55, wherein the viral vector is selected from the group consisting of AAV, adenovirus, lentivirus, retrovirus, poxvirus, baculovirus, herpes simplex virus, and a vaccinia virus.

**57.** The kit according to claim 56, wherein the viral vector is an AAV.

**58.** The kit according to claim 57, wherein the AAV is an AAV1, AAV2, AAV3, AAV4, AAV5, AAV6, AAV7, AAV8, AAV9, or AAVrh74 serotype.

**59.** The kit according to claim 55, wherein the viral vector is a pseudotyped AAV.

**60.** The kit according to claim 59, wherein the pseudotyped AAV is AAV2/8.

**61.** The kit of claim 59, wherein the pseudotyped AAV is AAV2/9.

FIG. 1

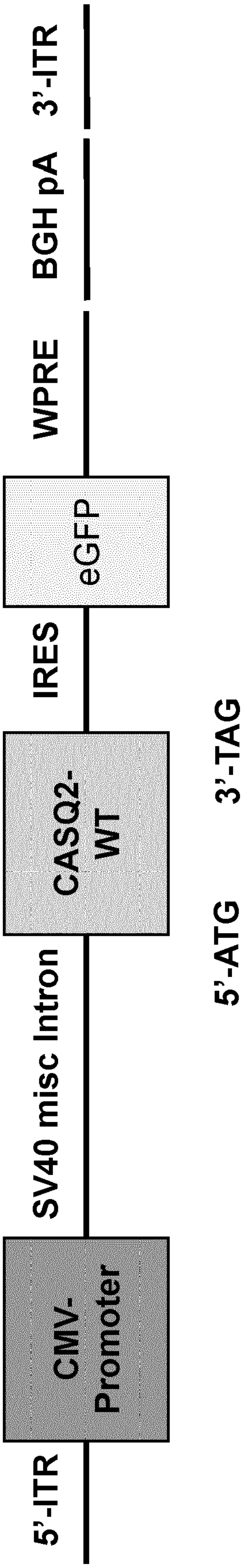
5'-ITR
CMV-
Promoter
SV40 misc Intron
CASQ2-
WT
5'-ATG
3'-TAG
IRES
eGFP
WPRE
BGH pA
3'-ITR

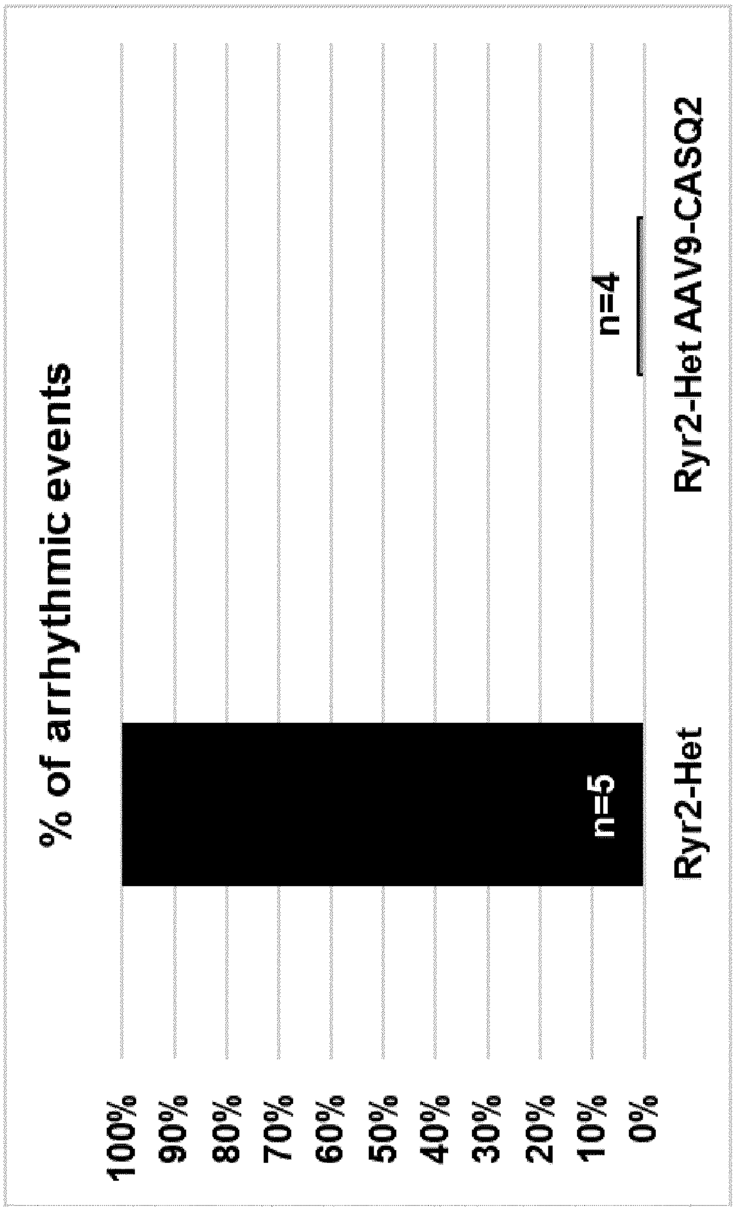
% of arrhythmic events
100%
90%
80%
70%
60%
50%
40%
30%
20%
10%
0%
n=5
n=4
Ryr2-Het
Ryr2-Het AAV9-CASQ2

FIG. 2

FIG. 3

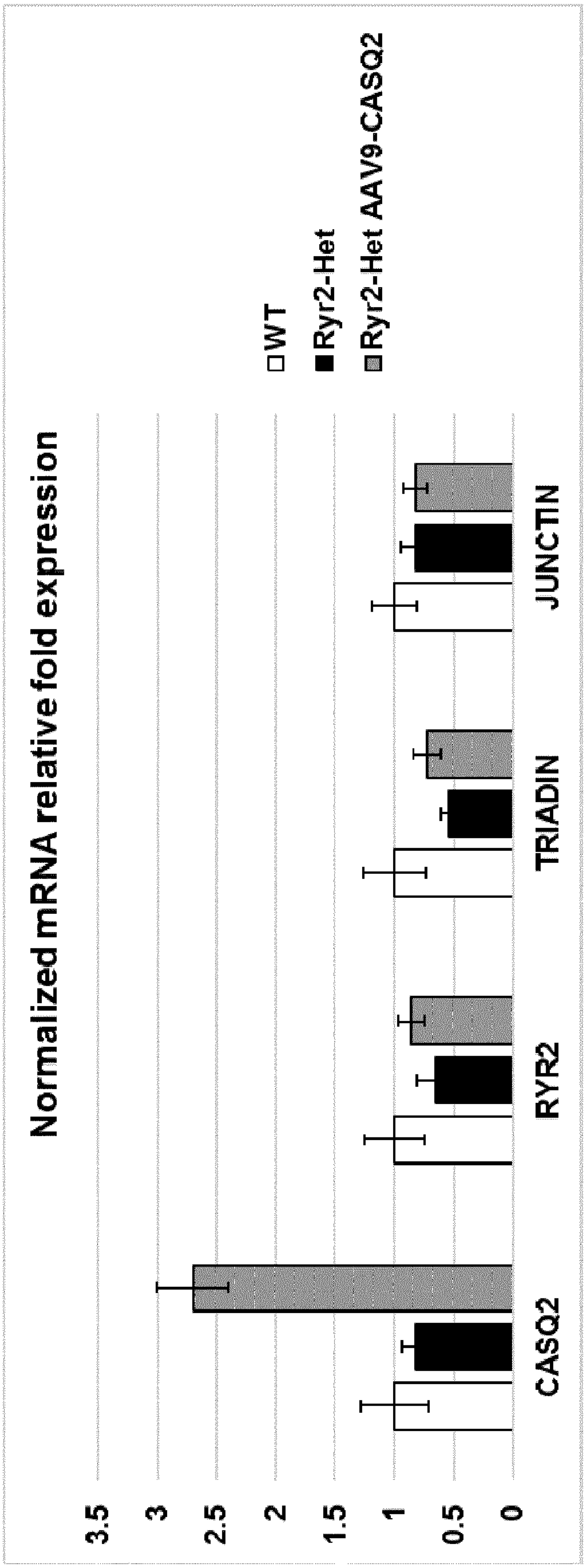
Normalized mRNA relative fold expression
3.5
3
2.5
2
1.5
1
0.5
0
CASQ2
RYR2
TRIADIN
JUNCTIN
WT
Ryr2-Het
Ryr2-Het AAV9-CASQ2

FIG. 4

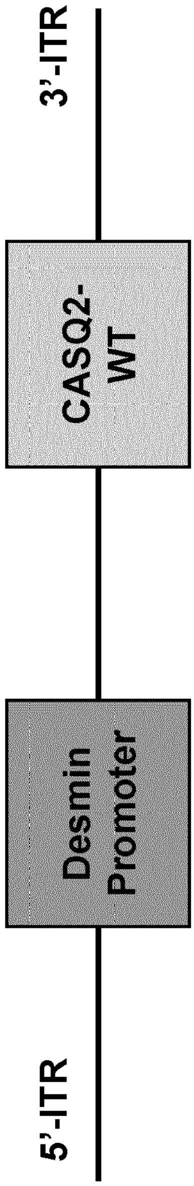

FIG. 5

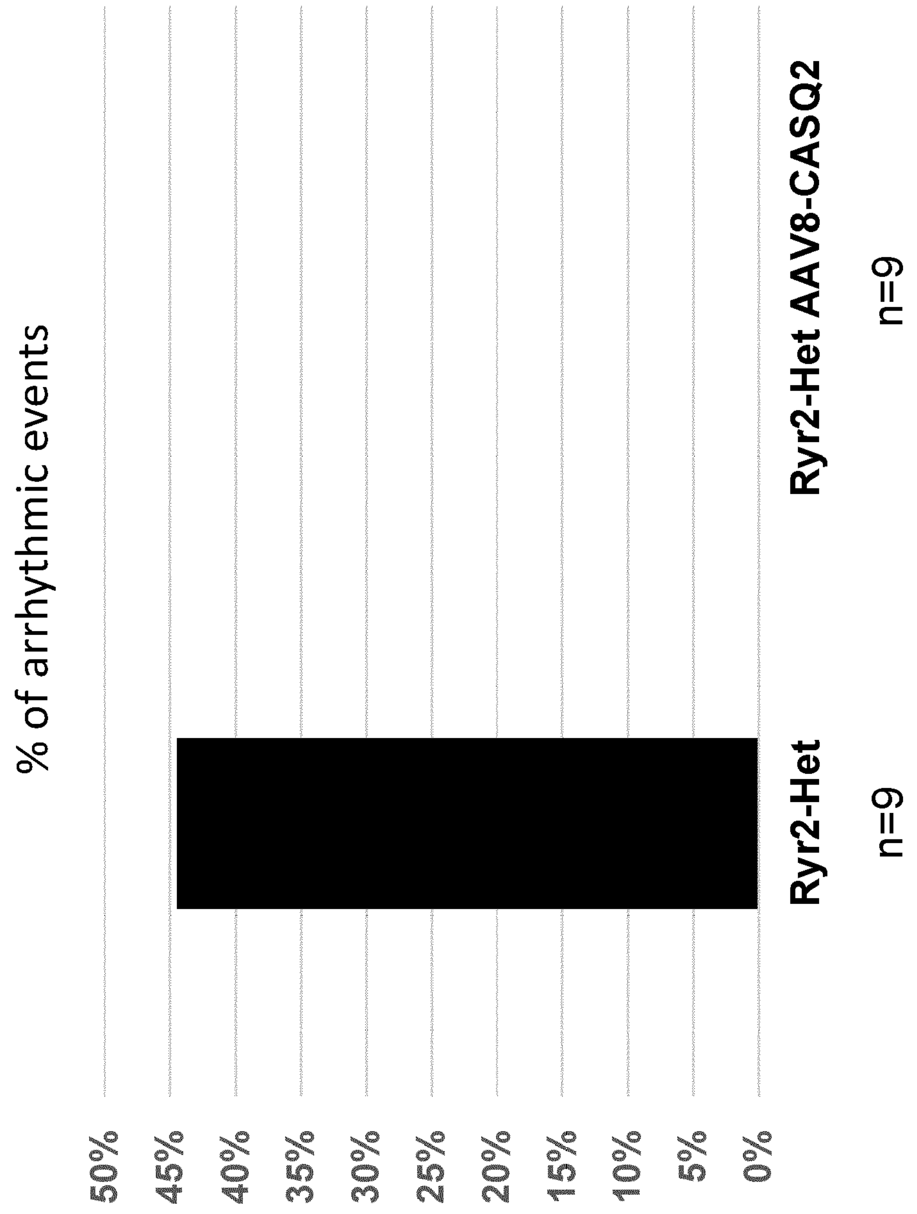
% of arrhythmic events
50%
45%
40%
35%
30%
25%
20%
15%
10%
5%
0%
Ryr2-Het
n=9
Ryr2-Het AAV8-CASQ2
n=9

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 199411026 A
- US 7741529 B
- US 5801030 A
- US 5173414 A
- US 5139941 A
- US 5863541 A
- US 5869305 A
- US 6057152 A
- US 6376237 B
- US 20100317114 A
- US 7442386 B
- US 20100227406 A
- WO 2010085699 A
- US 20050112764 A
- US 8697359 B
- US 8021867 B
- US 8445251 B
- US 6232068 B
- US 6268210 B
- US 5766960 A
- US 5143854 A
- US 5466468 A
- US 8859517 B


### Non-patent literature cited in the description


- **GAO et al.** *Journal of Biological Chemistry,* 1998, vol. 273, 6402-6409
- **JOHNSON et al.** *Methods Mol. Biol.,* 2002, vol. 173, 89-102
- **PRIORI et al.** *Circulation,* 2001, vol. 104 (II), 335
- **LAHAT et al.** *Am. J. Hum. Genet.,* 2001, vol. 69, 1378-1384
- **LIU et al.** *Circulation Research,* 2006, vol. 99, 292-298
- **HARLOW ; LANE.** Antibodies, A Laboratory Manual. Cold Spring Harbor Press, 1988
- **HARLOW ; LANE.** Using Antibodies, A Laboratory Manual. Cold Spring Harbor Press, 1999
- **GOEDDEL.** Gene Expression Technology: Methods in Enzymology. Academic Press, 1990, vol. 185
- **BERS.** *Nature,* 2002, vol. 415, 198-205
- **FRANZINI-ARMSTRONG.** *Ann. NY Acad. Sci.,* 2005, vol. 1047, 76-85
- **PIESKE et al.** *Circ. Res.,* 1999, vol. 85, 38-46
- **VENETUCCI et al.** *Nat. Rev. Cardiol.,* 2012, vol. 9, 561-75
- **CERRONE et al.** *Circulation Research,* 2005, vol. 96, e77-e82
- **COFFIN, J. M. et al.** Retroviridae: The viruses and their replication, In Fundamental Virology. Lippincott-Raven Publishers, 1996
- **TAI et al.** *J. Biomed. Sci.,* 2000, vol. 7, 279-291
- **MONAHAN ; SAMULSKI.** *Gene Delivery,* 2000, vol. 7, 24-30
- **RABINOWITZ et al.** *J. Virol.,* 2002, vol. 76, 791-801
- **BOWLES et al.** *J. Virol.,* 2003, vol. 77, 423-432
- **CHAO et al.** *Mol. Ther.,* 2000, vol. 2, 619-623
- **DAVIDSON et al.** *Proc. Natl. Acad. Sci. USA,* 2000, vol. 97, 3428-3432
- **XIAO et al.** *J. Virol.,* 1998, vol. 72, 2224-2232
- **HALBERT et al.** *J. Virol.,* 2000, vol. 74, 1524-1532
- **HALBERT et al.** *J. Virol.,* 2001, vol. 75, 6615-6624
- **AURICCHIO et al.** *Hum. Molec. Genet.,* 2001, vol. 10, 3075-3081
- **DUAN et al.** *J. Virol.,* 2001, vol. 75, 7662-7671
- **ZOLOTUKHIN et al.** *Methods,* 2002, vol. 28, 158-167
- **WU et al.** *J. Virol.,* 2000, vol. 74, 8635-45
- **SOONG et al.** *Nat. Genet.,* 2000, vol. 25, 436-439
- **KOLMAN ; STEMMER.** *Nat. Biotechnol.,* 2001, vol. 19, 423-428
- **CHU et al.** *Nucleic Acids Research,* 1987, vol. 15, 1311
- **DISTLER et al.** *Experimental Dermatology,* 2005, vol. 14, 315
- **SHAREI et al.** *Journal of Visualized Experiments,* 2013, vol. 81, e50980
- **DENNIG.** *Topics in Current Chemistry,* 2003, vol. 228, 227
- **GULICK et al.** *Current Protocols in Molecular Biology,* 1997, vol. 40, 1, , 9.2, , 9.2.1
- **RHODES et al.** *Methods in Cell Biology,* 2007, vol. 82, 309

- **QUINN et al.** Genetic Modification of Target Cells by Direct Delivery of Active Protein [abstract]. In: Methylation changes in early embryonic genes in cancer [abstract. *Proceedings of the 18th Annual Meeting of the American Society of Gene and Cell Therapy;,* 13 May 2015
- **HWANG et al.** *Nature Biotechnology,* 2013, vol. 31, 227
- **URNOV et al.** *Nature Reviews Genetics,* 2010, vol. 11, 636
- **JOUNG et al.** *Nature Reviews Molecular Cell Biology,* 2013, vol. 14, 49
- **SAMBROOK ; FRITCH ; MANIATIS.** Molecular Cloning, a Laboratory Manual. Cold Spring Harbor Press, 1989, vol. 10
- Current Protocols In Molecular Biology. 1995
- **MORTAZAVI et al.** *Nat. Methods,* 2008, vol. 5, 621-628
- **POLLACK et al.** *Nat. Genet.,* 1999, vol. 23, 41-46
- **GIBSON et al.** *Genome Res.,* 1996, vol. 6, 995-1001
- **HEID et al.** *Genome Res.,* 1996, vol. 6, 986-994
- **YATES et al.** *Annu. Rev. Biomed. Eng.,* 2009, vol. 11, 49-79
- Remington's Pharmaceutical Sciences. 1980
- **DENEGRI et al.** *Circulation,* 2014, vol. 129, 267381
- **CERRONE et al.** *M, Circulation Research,* 2005, vol. 96, e77-e82